# EUROPEAN PATENT APPLICATION

(11) **EP 3 771 713 A1**
(43) Date of publication of application: **03.02.2021**
(21) Application number: 19189653.9
(22) Date of filing: 01.08.2019
(51) Int. Cl.: C07D 401/12, A61P 25/28, A61K 31/473

(54) **CANNABINOID RECEPTOR SUBTYPE 2 STIMULATOR INHIBITING ACETYLCHOLINESTERASE AND BUTYRYLCHOLINESTERASE**

(71) Applicant: Julius-Maximilians-Universität Würzburg, 97070 Würzburg (DE)
(72) Inventor: SCHEINER, Matthias, 97070 Würzburg (DE); DOLLES, Dominik, 80339 München (DE); DECKER, Michael, 97074 Würzburg (DE)
(74) Representative: Dr. Gassner & Partner mbB

(57) **Abstract**

The invention concerns a compound according to formula 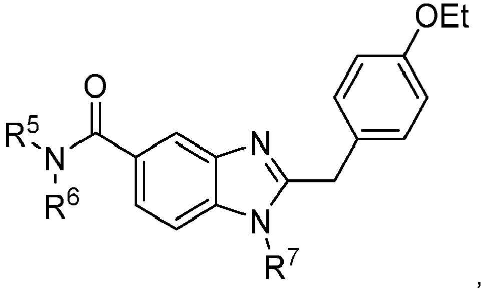 wherein R⁶ is H or an alkyl and one of R⁵ and R⁷ comprises or consists of linker-coupled tacrine residue 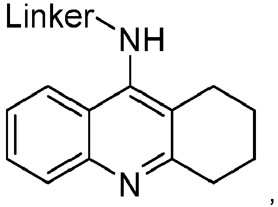 wherein the linker is coupled to the amino residue of the tacrine residue and couples the tacrine residue to the rest of the molecule, wherein the other one of R⁵ and R⁷ is H or an alkyl.

## Description

The invention concerns a compound inhibiting acetylcholinesterase (AChE) and butyrylcholinesterase (BChE) and stimulating cannabinoid receptor subtype 2 (CB₂R), the compound for use in the treatment of a disease, a use of that compound and a method for synthesizing that compound. The AChE, the BChE and the CB₂R can be independent from each other human AChE (*h*AChE), human BChE (*h*BChE) and human CB₂R (*h*CB₂R).

The exact molecular mechanisms, as well as the causes of an Alzheimer's Disease (AD) outbreak still remain unknown. However, a variety of biochemical changes during the pathological process are taking place. AD hallmarks are the formation of amyloid-beta (A*β*) plaques in the extracellular brain parenchyma, the abnormal hyperphosphorylation of the microtubule-associated tau-protein and the formation of neurofibrillary tangles (NFTs) within neurons. Both, the A*β* deposits and the NTFs, lead to a progressive loss of cholinergic neurons in the brain, memory deficits, and cognitive dysfunction. A*β* itself has been shown to exert cytotoxic effects on cultured neurons, to induce oxidative stress and modify ionic homeostasis. Furthermore, in post-mortem analysis of AD brain, increased expression of inflammatory mediators has been observed. The increase seems to be partly triggered by microglia activation. Microglia cells are activated by various stimuli, including misfolded A*β*, its precursor protein (APP), as well as misfolded tau. In the early stage of AD, stimulation leads to a neuroprotective M2 microglia phenotype and can therefore promote A*β* clearance via microglia's scavenger receptors (SRs) which hinder AD progression. However, it has been shown that there is a switch from the neuroprotective M2 to a more classically activated M1 phenotype at the later stage of AD. The M1 phenotype generally mediates defence from pathogens and tumor cells and it is characterized by the production of proinflammatory cytokines, such as TNF-α, IL-1β or reactive oxygen species (ROS), which are associated with the loss of neurons. Therefore, this switch can lead to an increased A*β* production, decreased A*β* clearance and, ultimately, to neuronal damage and progression of AD.

The human endocannabinoid system has been intensively investigated and two cannabinoid receptor (*h*CBR) subtypes have been discovered. The *h*CBR subtype 1 (*h*CB₁R) is the most abundant metabotropic receptor in the brain and it is involved in a variety of physiological processes. The *h*CB₂R is mainly expressed in peripheral immune cells and has been originally described as the peripheral CBR but later, it was found also in the central nervous system (CNS) where it is expressed on microglia and astrocytes. While the expression of the *h*CB₁R remains unchanged, the *h*CB₂R is abundantly and selectively expressed in neuritic plaque-associated astrocytes and microglia. In several *in vitro* and *in vivo* models of acute and chronic neurodegenerative disorders, the activation of the *h*CB₂R was shown to exert beneficial effects. *h*CB₂R agonists can suppress microglia activation and the production of neurotoxic factors as reactive oxygen species (ROS), nitric oxide (NO) and pro-inflammatory mediators (TNF-α and cytokines). Administration of a *h*CB₂R agonist to rats, previously and intracerebrally treated with A*β*₄₀, the forty amino acids long isoform of A*β*, promoted A*β* clearance, decreased secretion of proinflammatory mediators and, ultimately, led to increased synaptic plasticity, cognition, and memory.

The most established theory for the development of AD is the cholinergic hypothesis. Briefly, patients in AD suffer from an extensive loss of cholinergic neurons and cholinergic activity caused by reduced activity of choline acetylase. A rise of acetylcholine (ACh) level by inhibiting its enzymatic hydrolysis by acetylcholinesterase (AChE) proved to slow down the progression of AD and had a positive effect on patients' cognition and overall mental abilities in numerous clinical studies, both animal and human.

It has been found that BChE activity rises in response to reduced AChE activity in a later stage of AD and that BChE takes over the hydrolytic function of AChE. Therefore, inhibition of AChE is not sufficient to enhance ACh levels in this stage. Modern drugs for the treatment of AD target the two cholinesterases AChE and BChE. Besides the two reversible and selective AChE inhibitors donepezil and galantamine, there is also the carbamate based drug molecule rivastigmine, targeting both enzymes in a pseudo irreversible manner. Clinical trials showed that the approved drug molecules described above lack pharmacological action and effectivity in later stages of AD, whereas they slow down the progression of dementia in early stages.

Another inhibitor targeting both cholinesterases is the compound tacrine having the following formula:

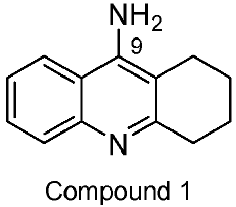

Tacrine, also designated compound 1 in the following, was the first approved drug for the treatment of AD but was withdrawn from the market because of its dose-dependent hepatotoxicity. From Chen, X. et al., J. Med. Chem. 2012, 55, pages 5231 to 5242 a codrug of tacrine and the hepatoprotective natural product silibinin showing neuro- and hepatoprotective effects *in vitro* and pro-cognitive and hepatoprotective effects *in vivo* is known.

WO 2008/074816 A1 concerns compounds with a combination of cannabinoid-CB₁ antagonism and acetylcholinesterase inhibition. The compound may have the formula A-[T]ₙ-B, wherein
A represents an essential structural element of any known cannabinoid-CB₁ antagonist containing at least two phenyl rings, independently optionally substituted with one or two substituents selected from the group consisting of halogen, methoxy, and trifluoromethyl, said essential structural element being attached to a hydrogen bond acceptor in said cannabinoid-CB₁ antagonist, wherein the hydrogen bond acceptor moiety is either a carbonyl group, a sulfonyl group, or a nitrogen or oxygen atom incorporated in a heteroaromatic ring structure,
T represents a linker consisting of a saturated or unsaturated linear carbon chain of 2-8 atoms, which carbon chain may be substituted with 1-5 substituents selected from methyl, ethyl, hydroxy, fluoro or amino, which carbon chain may contain an additional nitrogen atom, optionally substituted with a C₁₋₃ alkyl group, or which carbon chain may contain an additional oxygen or sulphur atom, or a carbonyl, sulfonyl, amide, sulfonamide, ureido, or aryl group, which aryl group is optionally substituted with 1-4 substituents selected from the group consisting of halogen, cyano, methyl, methoxy, trifluoromethyl, OCHF₂, OCF₃, SCF₃ or nitro,
B represents an essential structural element of any known acetylcholinesterase inhibitor and
n = 0 or 1.

Dolles, D. et al., J. Med. Chem. 2018, 61, pages 1646 to 1663 discloses the development of compounds inhibiting butyrylcholinesterase which compounds also act as ligands of human cannabinoid receptor 2. The authors describe that they merged pharmacophores for butyrylcholinesterase (BChE) and human cannabinoid receptor 2 (*h*CB₂R) into small molecules based on the known selective *h*CB₂R agonist having the following formula, also designated compound 2 in the following:

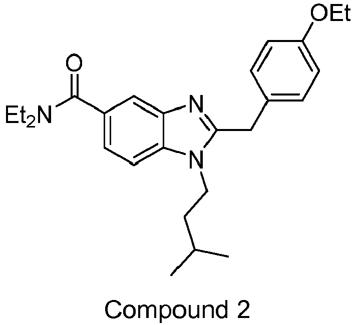

One well-balanced dual-acting and selective *h*BChE inhibitor/*h*CB₂R agonist showed superior *in vivo* activity over the above-mentioned known CB₂R agonist with regards to cognition improvement.

The problem to be solved by the present invention is to provide an alternative compound targeting AChE and BChE and stimulating CB₂R. Furthermore, that compound for use in the treatment of a disease, a use of that compound and a method for synthesizing that compound shall be given.

The problem is solved by the features of claims 1, 7, 8 and 9. Embodiments are subject-matter of claims 2 to 6 and 10 to 15.

According to the invention, a compound according to formula 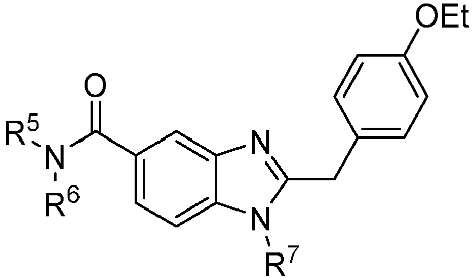 is provided, wherein R⁶ is H or an alkyl and one of R⁵ and R⁷ comprises or consists of linker-coupled tacrine residue 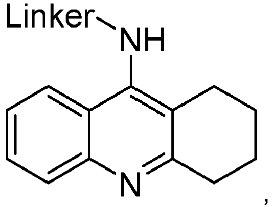 wherein the linker is coupled to the amino residue of the tacrine residue and couples the tacrine residue to the rest of the molecule, wherein the other one of R⁵ and R⁷ is H or an alkyl.

It is generally known that coupling two structures having defined features into a single molecule through a linker does only exceptionally result in a compound having the features of both single molecules. In the present case, the compound has inhibitory properties towards both ChEs and stimulating activity for the CB₂R. Defining a suitable connecting position for the linker chain was a crucial task, because an unsuitable connection between the two pharmacophore units can easily lead to a complete loss of activity or can reverse the behavior of the drug at the target, especially with regard to the *h*CB₂R, e. g., turn an agonist into an antagonist. The inventors found two possible attachment positions for a linker in the base molecule, namely one of R⁵ and R⁷ and one possible position for a linker in the tacrine residue. Connection of the two structures through these connection points lead to two sets of hybrid compounds having unexpected advantageous properties. In particular, it combines inhibition of AChE and BChE which inhibition is higher than that of tacrine with stimulation of CB₂R without the hepatotoxic effect of tacrine. Furthermore, animal experiments show that the compound is obviously able to cross the blood-brain barrier (BBB) though it has a molecular weight clearly above 600 and it is generally assumed that it is very difficult for molecules having a molecular weight above 500 to cross BBB. These new features could not have been expected.

The linker may comprise 2 to 8, in particular 2 to 6, C-residues. The linker may be unbranched or branched. The coupling may be at the ends of the linker that include the longest possible chain of atoms, preferably the longest possible chain of C-residues. The linker may consist solely of C- and H-atoms but may also comprise S-, O- or other atoms. In particular, the linker can comprise a disulfide group or at least one ether group. In an embodiment, the linker comprises two ether groups. The ether groups may be in a linker consisting of or comprising a polyethyleneglycol (PEG) residue. The structure and length of the linker influences the features of the compounds according to the invention. This is shown with respect to embodiments of the invention below.

The alkyl being R⁶ and/or the other one of R⁵ and R⁷ may be independent from each other methyl, ethyl, butyl, propyl, pentyl or hexyl. In an embodiment, the alkyl being the other one of R⁵ and R⁷ is an isoalkyl, in particular isopentyl.

In a specific embodiment, the compound is 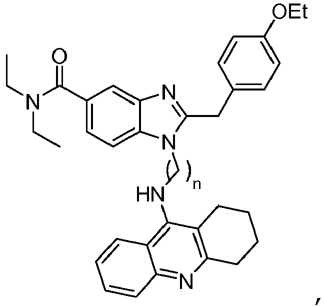 wherein n is 1 to 5 or 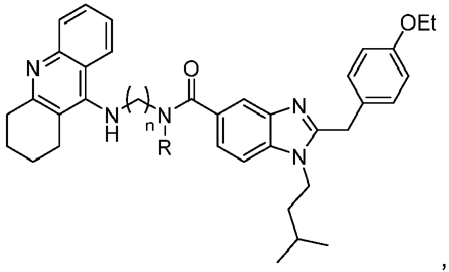 wherein n is 1 to 5 and R is H, methyl, ethyl, butyl, propyl, pentyl or hexyl or 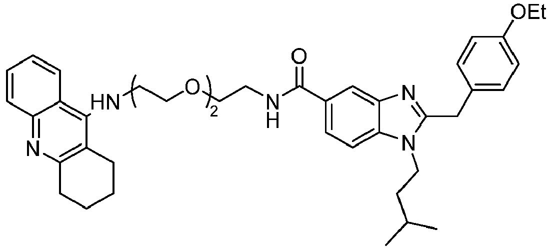 or 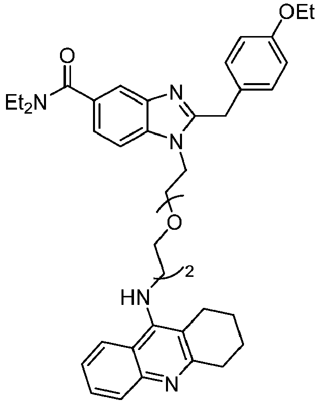 or

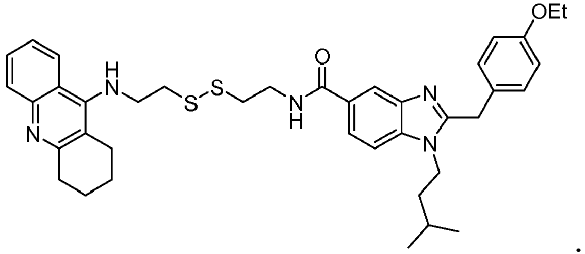

The invention further concerns a compound according to the invention for use in the treatment of Alzheimer's Disease in a mammal, in particular a human being. The treatment may occur by intravenous (i. v.), intradermal, subcutaneous, intramuscular or intraperitoneal (i. p.) injection of the compound according to the invention. Injection may be a depot injection, i. e. injection of a localized mass of the compound according to the invention, called a depot, from which depot the compound is gradually absorbed by surrounding tissue. The treatment may also occur by infiltration with the compound of the invention. Infiltration involves loading a volume of tissue with the compound of the invention by injection thus filling the interstitial space of that tissue with the compound.

A further aspect of the invention concerns the use of the compound according to the invention for inhibition of acetylcholinesterase (AChE) and/or butyrylcholinesterase (BChE) and/or stimulating cannabinoid receptor subtype 2 (CB₂R) *in vitro.* Furthermore, the compound according to the invention can be used for investigating Alzheimer's Disease in an animal model in which animal model at least one condition of Alzheimer's Disease is artificially induced, e. g. by intracerebroventricular injection of amyloid-beta fragment A*β*₂₅₋₃₅ in mice or other mammals. The condition can be any known symptom of AD, in particular forgetfulness.

Another aspect of the invention concerns a method for synthesizing a compound according to the invention comprising the following steps:
a) Reacting anthranilic acid (9) and cyclohexanone with an excess of phosphoroxylchloride to yield chloro-tetrahydroacridine,
b) dissolving chloro-tetrahydroacridine and reacting it with a diamine compound to give the corresponding tetrahydroacridine-diamine derivative,
c1) reacting 4-fluoro-3-nitrobenzoic acid with oxalylchloride and a catalytic amount of dimethylformamide (DMF) and then adding diethylamine to yield *N*,*N*-Diethyl-4-fluoro-3-nitrobenzamide,
d1) reacting *N*,*N*-Diethyl-4-fluoro-3-nitrobenzamide with the tetrahydroacridine-diamine derivative of step b) to yield 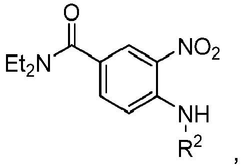 wherein R² is a residue of the tetrahydroacridine-diamine derivative,
e1) reducing the nitro moiety contained in the compound yielded at step d1) by means of a reducing agent to yield corresponding amine compound 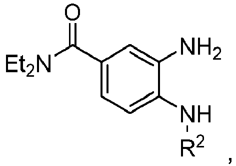
f1) activating 2-(4-Ethoxyphenyl)acetic acid with 2-(1H-Benzotriazol-1-yl)-1,1,3,3-tetramethyluronium-hexafluorophosphat (HBTU) and adding it to the amine compound obtained in step e1) to obtain corresponding amide compound 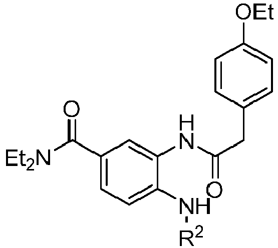 and
g1) cyclisation of the amide compound obtained in step f1) by means of acetic acid to yield compound 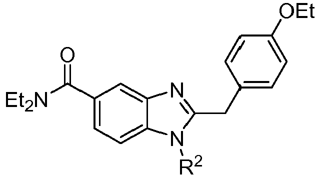 or
c2) adding a catalytical amount of H₂SO₄ to dissolved 4-fluoro-3-nitrobenzoic acid and basifying the resulting mixture after incubation by addition of triethylamine and 3-methylbutan-1-amine to yield ethyl 4-(isopentylamino)-3-nitrobenzoate,
d2) dissolving ethyl 4-(isopentylamino)-3-nitrobenzoate and reducing its nitro moiety with hydrogen over Pd/C to yield ethyl 3-amino-4-(isopentylamino)benzoate,
e2) activating 2-(4-Ethoxyphenyl)acetic acid with 2-(1H-Benzotriazol-1-yl)-1,1,3,3-tetramethyluronium-hexafluorophosphat (HBTU) and adding it to the ethyl 3-amino-4-(isopentylamino)benzoate to yield ethyl 3-(2-(4-ethoxyphenyl)acetamido)-4-(isopentylamino)benzoate,
f2) dissolving ethyl 3-(2-(4-ethoxyphenyl)acetamido)-4-(isopentylamino)benzoate in glacial acetic acid and basifying the resulting reaction mixture after incubation with NH₃ to yield ethyl 2-(4-ethoxybenzyl)-1-isopentyl-1H-benzo[d]imidazole-5-carboxylate,
g2) dissolving ethyl 2-(4-ethoxybenzyl)-1-isopentyl-1H-benzo[d]imidazole-5-carboxylate in solvent and adding an aqueous lithium hydroxide solution to yield 2-(4-ethoxybenzyl)-1-isopentyl-1H-benzo[d]imidazole-5-carboxylic acid after incubation and
h2) activating 2-(4-ethoxybenzyl)-1-isopentyl-1H-benzo[d]imidazole-5-carboxylic acid with 2-(1H-Benzotriazol-1-yl)-1,1,3,3-tetramethyluronium-hexafluorophosphat (HBTU) and adding the tetrahydroacridine-diamine derivative of step b) to yield 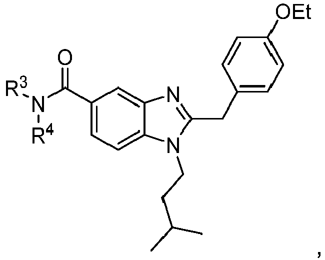 wherein R³ is a residue of the tetrahydroacridine-diamine derivative and R⁴ is H or an alkyl.

Step b) may further comprise acetylating the obtained tetrahydroacridine-diamine derivative for obtaining an amide and reducing this amide to obtain a secondary amine. The acetylating can be performed by incubation of the tetrahydroacridine-diamine derivative with acetic acid anhydride in dichloromethane. Reducing can be performed by incubation of the amide with LiAlH₄ in dry tetrahydrofuran (THF).

In an embodiment the diamine compound is NH₂-(CH₂)₂-NH₂, NH₂-(CH₂)₃-NH₂, NH₂-(CH₂)₄-NH₂, NH₂-(CH₂)₅-NH₂, NH₂-(CH₂)₆-NH₂, NH₂-[(CH₂)₂-O]₂-(CH₂)₂-NH₂ or NH₂-(CH₂)₂-S-S-(CH₂)₂-NH₂.

Steps a), b), e1), g1), c2) and/or f2) can comprise heating. Step c1) may comprise cooling and/or step d2) may comprise providing hydrogen at a pressure above atmospheric pressure, in particular above 2 bar, in particular above 3 bar, in particular above 4 bar, in particular above 5 bar, in particular above 6 bar, in particular above 7 bar, in particular above 8 bar, in particular above 9 bar. Usually, hydrogen is provided in step d2) at a pressure below 10 bar. In a specific embodiment, it is provided at 8 bar.

The reducing agent in step e1) can be SnCl₂. Dissolving in step b) may occur in hexanol. *N,N*-Diethyl-4-fluoro-3-nitrobenzamide can be reacted in step d1) in a solvent, in particular a mixture of THF and triethylamine. 4-fluoro-3-nitrobenzoic acid may be dissolved in step c2) in ethanol. In an embodiment dissolving of ethyl 4-(isopentylamino)-3-nitrobenzoate in step d2) occurs in methanol. Activating of 2-(4-Ethoxyphenyl)acetic acid with 2-(1H-Benzotriazol-1-yl)-1,1,3,3-tetramethyluronium-hexafluorophosphat (HBTU) in step f1) and/or e2) may occur in triethylamine. Dissolving of ethyl 2-(4-ethoxybenzyl)-1-isopentyl-1H-benzo[d]imidazole-5-carboxylate in step g2) can occur in tetrahydrofuran (THF).

Embodiments of the invention:
- Fig. 1: shows a reaction scheme for the synthesis of tacrine derivatives required for the synthesis of the compound according to the invention.
- Fig. 2: shows a reaction scheme for the synthesis of compounds according to the invention.
- Fig. 3: shows a reaction scheme for the synthesis of further compounds according to the invention.
- Fig. 4: shows a diagram of the expression of cAMP-regulated genes Macrophage migration Inhibitory Factor (MIF) and Signal Transducer and Activator of Transcription (STAT-3) as a result of activation of *h*CB₂R by compounds 2, 3e and 4a according to the invention.
- Fig. 5 a-d: show the effects of compounds 2, 3e and 4a on microglial activation in cells of murine microglial cell line N9 previously activated by lipopolysaccharide (LPS) in western blots and diagrams.
- Fig. 6: shows the effects of compounds 4e and 8 on cells of murine hippocampal neuron cell line HT-22 with respect to neurotoxicity and neuroprotectivity in diagrams.
- Fig. 7: shows a protocol of the experimental treatment and testing of mice.
- Fig. 8: shows the effect of compounds according to the invention on A*β*₂₅₋₃₅-induced learning impairments in mice.

### Synthesis of compounds

### Principle of synthesis

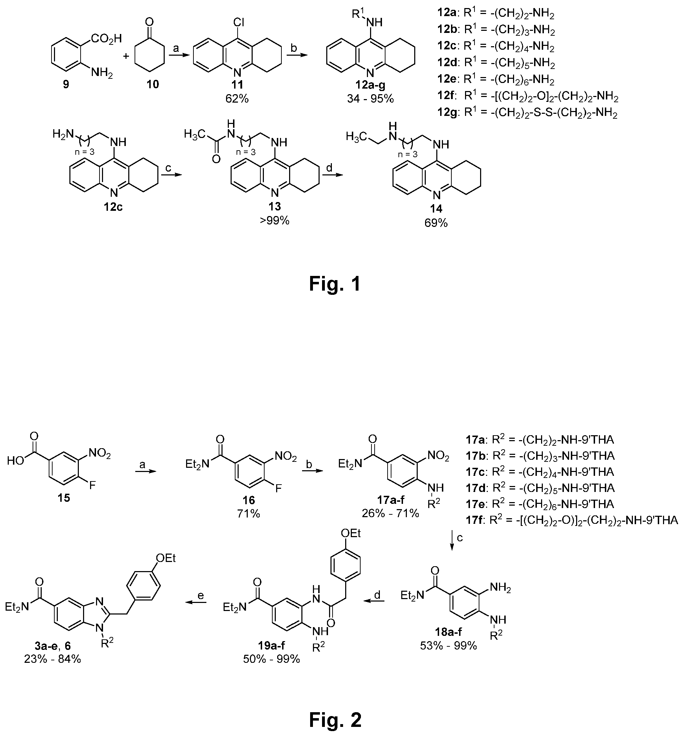

Tacrine-amine derivatives 12a-g and 14 for later coupling to the benzimidazole core were synthesized as illustrated in Fig. 1. Anthranilic acid 9 and cyclohexanone 10 were heated up with an excess of phosphoroxylchloride to yield the tetrahydroacridine derivative 11. By substitution with the corresponding diamine in excess, the tacrine-amine derivatives 12a-g were obtained. For the synthesis of derivative 14, the primary amine of tacrine amine derivative 12c was acetylated to form amide 13 in quantitative yield. In the next step, the amide was reduced to the secondary amine 14 in the presence of lithium aluminum hydride. Reagents and conditions of the reactions illustrated in Fig. 1 were as follows: (a) POCl3, reflux; (b) respective diamine, hexanol, reflux; (c) Ac2O, r.t.; (d) LiAlH4, THF, reflux; (yields are given in %; r.t. = room temperature)

Hybrids 3a-e and 6 were synthesized as illustrated in Fig. 2. Synthesis of derivatives 3a-e and 6 started with the 4-fluoro-3-nitrobenzoic acid 15. To form the diethylamide 16, oxalylchloride and a catalytic amount of DMF was used, then diethylamine added. In the next step, the fluorine atom was substituted with the respective amine derivative 12a-f to form compounds 17a-f, the reaction gave good to quantitative yield. The nitro moiety was then reduced with tin(II)-chloride dihydrate to obtain the anilinic amines 18a-f, which were directly used for the next step. 2-(4-Ethoxyphenyl)acetic acid was activated with HBTU and added to amines 18a-f. In the last step, the amides 19a-f were cyclized in acetic acid to yield compounds 3a-e and 6. Reagents and conditions of the reactions illustrated in Fig. 2 were as follows: (a) HNEt₂, cat. DMF, (COCl)₂, CH₂Cl₂ 0 °C, grow to r.t.; (b) respective amine 12a-f, THF, NEt₃, r.t.; (c) SnCl₂•2 H₂O, EtOH, reflux; (d) 2-(4-ethoxyphenyl)acetic acid, HBTU, NEt₃, DMF, r.t.; (e) AcOH, reflux; (yields are given in %; Et = ethyl).

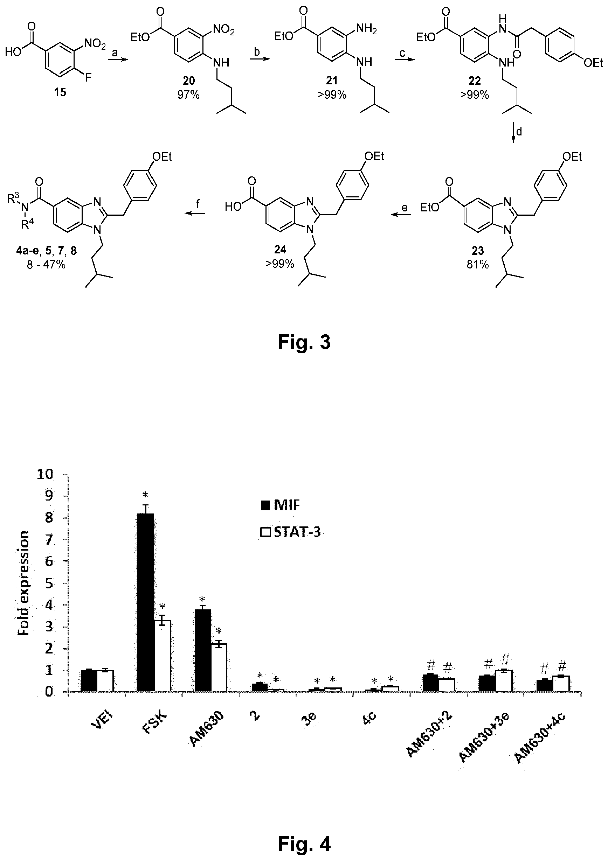

Hybrids 4a-e, 5, 7 and 8 were synthesized as illustrated in Fig. 3. 4-Fluoro-3-nitrobenzoic acid 15 served as starting material for derivatives 4a-e, 5, 7 and 8. In the first step, the ethyl ester was formed as a protection group, and the fluorine atom was substituted with 3-methylbutan-1-amine to yield substituted ester 20 in a good yield. In the next step, the nitro moiety was reduced with hydrogen over Pd/C to yield amine 21, which was then directly reacted with HBTU activated 2-(4-ethoxyphenyl)acetic acid to form amide 22. Benzimidazole 23 was formed by stirring 22 in acetic acid under reflux. The ester was hydrolyzed under basic conditions. In the last step, acid 24 was activated with HBTU and reacted with the respective amines 12a-g and 14 to yield hybrids 4a-e, 5, 7 and 8.

Reagents and conditions of the reactions illustrated in Fig. 3 were as follows: (a) I) cat. H₂SO₄ (97%), EtOH, reflux II) NEt₃ 3-methylbutan-1-amine, r.t.; (b) cat. Pd/C (10%), MeOH, H₂ atm., 8 bar, r.t.; (c) 2-(4-ethoxyphenyl)acetic acid, HBTU, NEt₃, DMF, r.t.; (d) AcOH, reflux; (e) LiOH, H₂O, reflux; (f) respective amine 12a-g, 14, HBTU, NEt₃, DMF, r.t.; (yields are given in %).

### General Procedures

*General procedure I for nucleophilic substitution of aryl chloride by a diamine (for compounds 12a-g).* The chloride compound 11 was dissolved in hexanol and the corresponding diamine was added. The mixture stirred for 24 h under reflux conditions argon atmosphere. The solvent was evaporated under high vacuum using an evaporator. The residue was dissolved in CH₂Cl₂ and extracted with water. The organic layer was dried over anhydrous MgSO₄ and the solvent was removed *in vacuo.*

*General procedure II for aromatic substitution fluoride by amines (for compounds 17a-f, 20).* The respective fluoride compound and the respective amine were dissolved in THF and NEt₃ was added. The mixture stirred at room temperature overnight. The solvent was then removed *in vacuo* and the residue was dissolved in CH₂Cl₂ and washed with water. The organic layers were combined, dried over anhydrous MgSO₄ and the solvent was removed *in vacuo.*

*General procedure III for reduction of an aromatic nitro moiety with stannous chloride dihydrate (for compounds 18a-f).* The respective nitro compound was dissolved in ethanol and SnCl₂·2 H₂O was added. The mixture was stirred under reflux conditions under argon atmosphere for 5 h. Then, the mixture was basified with NH_{3(aq)} (25%) and the precipitate was filtered off by suction. The filtrate was concentrated *in vacuo.* The residue was dissolved in CH₂Cl₂ and washed with 1 M NaOH. The organic layers were combined, dried over anhydrous MgSO₄ and the solvent was removed *in vacuo.* The product was directly used in the next reaction, without further purification and just characterized by mass spectrometry.

*General procedure IV for amide formation with HBTU activated ester (for compounds 19a-f, 22)*.The respective acid was dissolved in DMF then NEt₃ and HBTU were added. This mixture was added to a solution of the respective amine in DMF. The mixture stirred over night at room temperature and the solvent was then removed *in vacuo.* The residue was dissolved in CH₂Cl₂ and washed with a saturated NaHCO₃ solution. The organic layers were combined, dried over anhydrous MgSO₄ and the solvent was removed *in vacuo.*

*General procedure V for ring-closure to benzimidazole (for compounds 3a-e, 6,* 23). The respective amide was dissolved in glacial acetic acid. The mixture stirred depending on the reaction progress 2-6 h under reflux conditions and was then concentrated *in vacuo.* The residue was basified with NH_{3(aq)} (25%) and extracted with CH₂Cl₂. The organic layers were combined, dried over anhydrous MgSO₄ and concentrated *in vacuo.*

### Detailed procedures

*9-Chloro-1,2,3,4-tetrahydroacridine (11)*. 2-Aminobenzoic acid 9 (1.00 g, 7.29 mmol) and cyclohexanon 10 (883 µL, 8.53 mmol) were combined in a flask and cooled down to 0 °C. Then POCl₃ (8.00 mL) was slowly added. Afterwards the mixture stirred for 3 h under reflux conditions and then concentrated to a slurry *in vacuo.* The slurry was dissolved in ethyl acetate and neutralized with aqueous sodium hydroxide. The mixture was extracted with ethyl acetate. The organic layers were combined, dried over anhydrous MgSO₄ and the solvent was removed *in vacuo.* The crude product was purified by recrystallization from acetone. The product 11 (988 mg, 4.54 mmol, 62%) was obtained as a brown solid.

*N¹-(1,2,3,4-Tetrahydroacridin-9-yl)ethane-1,2-diamine (12a).* The reaction was carried out according to general procedure I using 9-chloro-1,2,3,4-tetrahydroacridine 11 (800 mg, 3.67 mmol) and ethane-1,2-diamine (1.23 mL 18.35 mmol). The product 12a (557 mg, 2.31 mmol, 63%) was obtained as a brown oil.

*N¹-(1,2,3,4-Tetrahydroacridin-9-yl)propane-1,3-diamine (12b).* The reaction was carried out according to general procedure I using 9-chloro-1,2,3,4-tetrahydroacridine 11 (600 mg, 2.76 mmol) and propane-1,3-diamine (1.15 mL, 13.8 mmol). The product 12b (530 mg, 1.97 mmol, 80%) was obtained as a brown oil (417 mg, 1.63 mmol, 59%).

*N¹-(1,2,3,4-Tetrahydroacridin-9-yl)butane-1,4-diamine (12c).* The reaction was carried out according to general procedure I using 9-chloro-1,2,3,4-tetrahydroacridine 11 (535 mg, 2.45 mmol) and butane-1,4-diamine (1.08 g, 12.3 mmol). The product 12c (530 mg, 1.97 mmol, 80%) was obtained as a brown oil.

*N¹-(1,2,3,4-Tetrahydroacridin-9-yl)pentane-1,5-diamine (12d).* The reaction was carried out according to general procedure I using 9-chloro-1,2,3,4-tetrahydroacridine 11 (600 mg, 2.76 mmol) and pentane-1,5-diamine (1.62 mL, 13.8 mmol). The product 12d (427 mg, 1.51 mmol, 55%) was obtained as a brown oil.

*N¹-(1,2,3,4-Tetrahydroacridin-9-yl)hexane-1,6-diamine (12e)*. The reaction was carried out according to general procedure I using 9-chloro-1,2,3,4-tetrahydroacridine 11 (500 mg, 2.30 mmol) and hexane-1,6-diamine (1.50 mL, 11.5 mmol). The product 12e was obtained as a brown oil (343 mg, 1.15 mmol, 50%).

*N-(2-(2-(2-Aminoethoxy)ethoxy)ethyl)-1,2,3,4-tetrahydroacridin-9-amine (12f).* The reaction was carried out according to general procedure I using 9-chloro-1,2,3,4-tetrahydroacridine 11 (600 mg, 2.76 mg) and 2,2'-(ethane-1,2-diylbis(oxy))bis(ethan-1-amine) (2.00 mL, 13.8 mmol). The product 12f (864 mg, 2.62 mmol, 95%) was obtained as a brown oil.

*N-(2-((2-Aminoethyl)disulfaneyl)ethyl)-1,2,3,4-tetrahydroacridin-9-amine (12g).* 2,2'-Dithiobis(ethylamine)dihydrochloride (608 mg, 2.70 mmol) was suspended in hexanol and NEt₃ (1.49 mL, 10.8 mmol) was added. The reaction vessel was closed and stirred for 30 min at 160 °C. Then 9-chloro-1,2,3,4-tetrahydroacridine 11 (117 mg, 0.54 mg) was added and the mixture stirred for further 12 h at 160 °C. The solvent was removed under high vacuum at 60 °C. The crude product was purified using RP flash chromatography to yield 12g (80 mg, 0.33 mmol, 44%) as a pale-yellow oil.

*N-(4-((1,2,3,4-Tetrahydroacridin-9-yl)amino)butyl)acetamide (13). N*¹-(1,2,3,4-Tetrahydroacridin-9-yl)butane-1,4-diamine 12c (51.0 mg, 0.19 mmol) was dissolved in CH₂Cl₂ and acetic acid anhydride (359 µL, 3.80 mmol) was added at room temperature. After stirring for 5 min, the mixture was basified by washing with a saturated NaHCO₃ solution. The organic layer was dried over anhydrous MgSO₄ and concentrated *in vacuo.* The product 13 (59.2 mg, 0.19 mmol, quant.) was obtained as a pale-yellow oil.

*N¹-Ethyl-N⁴-(1,2,3,4-tetrahydroacridin-9-yl)butane-1,4-diamine. N*-(4-((1,2,3,4-Tetrahydroacridin-9-yl)amino)butyl)acetamide 13 (218 mg, 0.70 mmol) was dissolved in dry THF. LiAlH₄ (133 mg, 3.50 mmol) was added, the mixture stirred for 48 h under reflux conditions and then NH_{3(aq)} (25%) was carefully added to the mixture on ice. The mixture was concentrated *in vacuo,* dissolved in ethyl acetate and washed with a mixture of water/NH_{3(aq)} (25%) (1:1). The combined organic layers were dried over anhydrous MgSO₄ and concentrated *in vacuo.* The crude product was purified by column chromatography (4:1:0.1 CH₂Cl₂:MeOH:NH_{3(aq)} (25%)). The product 14 (144 mg, 0.48 mmol, 69%) was obtained as a yellow oil.

*N,N-Diethyl-4-fluoro-3-nitrobenzamide* (16). 4-Fluoro-3-nitrobenzoic acid 15 (1.00 g, 5.4 mmol) was dissolved in CH₂Cl₂ and cooled down to 0 °C. A catalytic amount of *N*,*N*-dimethylformamide was added and oxalylchloride (2.31 mL, 27.01 mmol) was added dropwise. The mixture stirred for 10 min at 0 °C and was then allowed to come to room temperature and stirred for 1 h. The mixture was then evaporated, the residue dissolved in CH₂Cl₂ and cooled down to 0 °C. Then a mixture of HNEt₂ (621 µL, 1.1 mmol) and NEt₃ (2.24 mL, 16.2 mmol) in CH₂Cl₂ was slowly added to the cold mixture. After stirring for 3 h at room temperature, the mixture was washed with water. The combined organic layers were dried over anhydrous MgSO₄ and concentrated *in vacuo.* The Product 16 (918 mg, 3.82 mmol, 71%) was obtained as a yellow oil.

*N, N-Diethyl-3-nitro-4-((2-((1,2,3,4-tetrahydroacridin-9-yl)amino)ethyl)amino)benzamide (17a).* The reaction was carried out according to general procedure II using *N*,*N*-diethyl-4-fluoro-3-nitrobenzamide 16 (351 mg, 1.46 mmol), 12a (388 mg, 1.61 mmol) and NEt₃ (304 µL, 2.19 mmol). The crude product was purified by column chromatography (20:1:0.1 CH₂Cl₂:MeOH:NH_{3(aq)} (25%)). and the product 17a (475 mg, 1.03 mmol, 71%) was obtained as a yellow oil.

*N, N-Diethyl-3-nitro-4-((3-((1,2,3,4-tetrahydroacridin-9-yl)amino)propyl)amino)benzamide (17b).* The reaction was carried out according to general procedure II using *N*,*N*-diethyl-4-fluoro-3-nitrobenzamide 16 (173 mg, 0.72 mmol), 12b (165 mg, 0.65 mmol) and NEt₃ (136 µL, 0.98 mmol). The crude product was purified by flash chromatography (gradient CH₂Cl₂, MeOH) and the product 17b (110 mg, 0.23 mmol, 36%) was obtained as a brown oil.

*N, N-Diethyl-3-nitro-4-((4-((1,2,3,4-tetrahydroacridin-9-yl)amino)butyl)amino)benzamide (17c).* The reaction was carried out according to general procedure II using *N*,*N*-diethyl-4-fluoro-3-nitrobenzamide 16 (430 mg, 1.79 mmol), 12c (439 mg, 1.63 mmol) and NEt₃ (340 µL, 2.45 mmol). The crude product was purified by flash chromatography (gradient CH₂Cl₂, MeOH) and the product 17c (212 mg, 0.43 mmol, 26%) was obtained as a brown oil.

*N, N-Diethyl-3-nitro-4-((5-((1,2,3,4-tetrahydroacridin-9-yl)amino)pentyl)amino)benzamide (17d).* The reaction was carried out according to general procedure II using *N*,*N*-diethyl-4-fluoro-3-nitrobenzamide 16 (221 mg, 0.92 mmol), 12d (237 mg, 0.84 mmol) and NEt₃ (173 µL, 1.26 mmol). The crude product was purified by flash chromatography (gradient CH₂Cl₂, MeOH) and the product 17d (160 mg, 0.32 mmol, 38%) was obtained as a brown oil.

*N, N-Diethyl-3-nitro-4-((6-((1,2,3,4-tetrahydroacridin-9-yl)amino)hexyl)amino)benzamide (17e)*. The reaction was carried out according to general procedure II using *N*,*N*-diethyl-4-fluoro-3-nitrobenzamide 16 (341 mg, 1.42 mmol), 12e (458 mg, 1.56 mmol) and NEt₃ (297 µL, 2.13 mmol). The product 17e (230 mg, 0.44 mmol, 31%) was obtained as a brown oil and used in the next reaction step without further purification.

*N,N-Diethyl-3-nitro-4-((2-(2-(2-((1,2,3,4-tetrahydroacridin-9-yl)amino)ethoxy)ethoxy)ethyl) amino)benzamide (17f).* The reaction was carried out according to general procedure II using *N*,*N*-diethyl-4-fluoro-3-nitrobenzamide 16 (475 mg, 1.98 mmol), 12f (718 mg, 2.18 mmol) and NEt₃ (410 µL, 2.97 mmol). The crude product was purified by flash chromatography (gradient CH₂Cl₂/MeOH) and product 17f (575 mg, 1.05 mmol, 53%) was obtained as a brown oil and used in the next reaction step without further purification.

*3-Amino-N,N-diethyl-4-((2-((1,2,3,4-tetrahydroacridin-9-yl)amino)ethyl)amino)benzamide (18a).* The reaction was carried out according to general procedure III using *N,N*-Diethyl-3-nitro-4-((2-((1,2,3,4-tetrahydroacridin-9-yl)amino)ethyl)amino)benzamide 17a (475 mg, 1.03 mmol) and SnCl₂·2 H₂O (1.44 g, 6.39 mmol). The product 18a (325 mg, 0.75 mmol, 73%) was obtained as a colorless oil and was directly used for the next reaction without purification.

*3-Amino-N,N-diethyl-4-((3-((1,2,3,4-tetrahydroacridin-9-yl)amino)propyl)amino)benzamide (18b).* The reaction was carried out according to general procedure III using *N*,*N*-diethyl-3-nitro-4-((3-((1,2,3,4-tetrahydroacridin-9-yl)amino)propyl)amino)benzamide 17b (110 mg, 0.23 mmol) and SnCl₂·2 H₂O (323 mg, 1.43 mmol). The product 18b (89.1 mg, 0.20 mmol, 87%) was obtained as a colorless oil and was directly used for the next reaction without purification.

*3-Amino-N,N-diethyl-4-((4-((1,2,3,4-tetrahydroacridin-9-yl)amino)butyl)amino)benzamide (18c).* The reaction was carried out according to general procedure III using *N*,*N*-diethyl-3-nitro-4-((4-((1,2,3,4-tetrahydroacridin-9-yl)amino)butyl)amino)benzamide 17c (212 mg, 0.43 mmol) and SnCl₂·2 H₂O (602 mg, 2.67 mmol). The product 18c (154 mg, 0.34 mmol, 79%) was obtained as a colorless oil and directly used for the next reaction without purification.

*3-Amino-N,N-diethyl-4-((5-((1,2,3,4-tetrahydroacridin-9-yl)amino)pentyl)amino)benzamide (18d)*. The reaction was carried out according to general procedure III using *N*,*N*-diethyl-3-nitro-4-((5-((1,2,3,4-tetrahydroacridin-9-yl)amino)pentyl)amino)benzamide 17d (236 mg, 0.47 mmol) and SnCl₂·2 H₂O (657 mg, 2.91 mmol). The product 18d (222 mg, 0.47 mmol, quant.) was obtained as a colorless oil, and directly used for the next reaction without purification.

*3-Amino-N,N-diethyl-4-((6-((1,2,3,4-tetrahydroacridin-9-yl)amino)hexyl)amino)benzamide (18e).* The reaction was carried out according to general procedure III using *N*,*N*-diethyl-3-nitro-4-((6-((1,2,3,4-tetrahydroacridin-9-yl)amino)hexyl)amino)benzamide 17e (230 mg, 0.44 mmol) and SnCl₂·2 H₂O (616 mg, 2.73 mmol). The product 18e (140 mg, 0.29 mmol, 66%) was obtained as a colorless oil and directly used for the next reaction without purification. ESI: *m*/*z* calcd for C₃₀H₄₂N₅O [M+2H]²⁺: 244.68; found: 244.65; Retention time: 7.93 min.

*3-Amino-N,N-diethyl-4-((2-(2-(2-((1,2,3,4-tetrahydroacridin-9-yl)amino)ethoxy)ethoxy)ethyl) amino)benzamide (18f).* The reaction was carried out according to general procedure III using N,N-diethyl-3-nitro-4-((2-(2-(2-((1,2,3,4-tetrahydroacridin-9-yl)amino)ethoxy)ethoxy)ethyl) amino)benzamide 17f (575 mg, 1.05 mmol) and SnCl₂·2 H₂O (1.47 g, 8.51 mmol). The product 18f (290 mg, 0.56 mmol, 53%) was obtained as a colorless oil and directly used for the next reaction without purification.

*3-(2-(4-Ethoxyphenyl)acetamido)-N,N-diethyl-4-((2-((1,2,3,4-tetrahydroacridin-9-yl)amino)ethyl)amino)benzamide (19a).* The reaction was carried out according to general procedure IV using 3-Amino-*N*,*N*-diethyl-4-((2-((1,2,3,4-tetrahydroacridin-9-yl)amino)ethyl)amino)benzamide 18a (325 mg, 0.75 mmol), 2-(4-ethoxyphenyl)acetic acid (30 mg, 0.83 mmol), HBTU (313 mg, 0.83 mmol) and triethylamine (156 µL, 1.13 mmol). The product 19a (258 mg, 0.43 mmol, 57%) was obtained as an orange/brown oil.

*3-(2-(4-Ethoxyphenyl)acetamido)-N,N-diethyl-4-((3-((1,2,3,4-tetrahydroacridin-9-yl)amino)propyl)amino)benzamide (19b).* The reaction was carried out according to general procedure IV using 3-amino-*N*,*N*-diethyl-4-((3-((1,2,3,4-tetrahydroacridin-9-yl)amino)propyl)amino)benzamide 18b (154 mg, 0.34 mmol), 2-(4-ethoxyphenyl)acetic acid (136 mg, 0.37 mmol), HBTU (140 mg, 0.37 mmol) and triethylamine (70.6 µL, 0.51 mmol). The product 19b (104 mg, 0.17 mmol, 50%) was obtained as an orange/brown oil.

*3-(2-(4-Ethoxyphenyl)acetamido)-N,N-diethyl-4-((4-((1,2,3,4-tetrahydroacridin-9-yl)amino)butyl)amino)benzamide (19c)*. The reaction was carried out according to general procedure IV using 3-amino-*N*,*N*-diethyl-4-((4-((1,2,3,4-tetrahydroacridin-9-yl)amino)butyl)amino)benzamide 18c (139 mg, 0.30 mmol), 2-(4-ethoxyphenyl)acetic acid (121 mg, 0.33 mmol), HBTU (125 mg, 0.33 mmol) and triethylamine (62.3 µL, 0.45 mmol). The product 19c (166 mg, 0.27 mmol, 90%) was obtained as a brown oil.

*3-(2-(4-Ethoxyphenyl)acetamido)-N,N-diethyl-4-((5-((1,2,3,4-tetrahydroacridin-9-yl)amino)pentyl)amino)benzamide (19d)*. The reaction was carried out according to general procedure IV using 3-amino-*N*,*N*-diethyl-4-((5-((1,2,3,4-tetrahydroacridin-9-yl)amino)pentyl)amino)benzamide 19d (222 mg, 0.47 mmol,) 2-(4-ethoxyphenyl)acetic acid (191 mg, 0.52 mmol), HBTU (197 mg, 0.52 mmol) and triethylamine (98.4 µL, 0.71 mmol). The product 19d (299 mg, 0.47 mmol, quant.) was obtained as a brown oil.

*3-(2-(4-Ethoxyphenyl)acetamido)-N,N-diethyl-4-((6-((1,2,3,4-tetrahydroacridin-9-yl)amino)hexyl)amino)benzamide (19e)*. The reaction was carried out according to general procedure IV using 3-amino-*N*,*N*-diethyl-4-((6-((1,2,3,4-tetrahydroacridin-9-yl)amino)hexyl)amino)benzamide 18e (140 mg, 0.29 mmol), 2-(4-ethoxyphenyl)acetic acid (52.3 mg, 0.29 mmol), HBTU (133 mg, 0.35 mmol) and triethylamine (61.0 µL, 0.44 mmol). The product 19e (142 mg, 0.22 mmol, 76%) was obtained as a brown oil.

*3-(2-(4-Ethoxyphenyl)acetamido)-N,N-diethyl-4-((2-(2-(2-((1,2,3,4-tetrahydroacridin-9-yl)amino)ethoxy)ethoxy)ethyl)amino)benzamide (19f).* The reaction was carried out according to general procedure IV using 3-amino-*N,N*-diethyl-4-((2-(2-(2-((1,2,3,4-tetrahydroacridin-9-yl)amino)ethoxy)ethoxy)ethyl)amino)benzamide 18f (290 mg, 0.56 mmol) 2-(4-ethoxyphenyl)acetic acid (122 mg, 0.62 mmol), HBTU (235 mg, 0.62 mmol) and triethylamine (116 µL, 0.84 mmol). The product 19f (398 mg, 0.58 mmol, quant.) was obtained as a brown oil.

*2-(4-Ethoxybenzyl)-N,N-diethyl-1-(2-((1,2,3,4-tetrahydroacridin-9-yl)amino)ethyl)-1H-benzo[d]imidazole-5-carboxamide (3a)*. The reaction was carried out according to general procedure V using 3-(2-(4-Ethoxyphenyl)acetamido)-*N,N*-diethyl-4-((2-((1,2,3,4-tetrahydroacridin-9-yl)amino)ethyl)amino)benzamide 19a (258 mg, 0.43 mmol). The crude product was purified by column chromatography (20:1:0.1 CH₂Cl₂:MeOH:NH_{3(aq)} (25%)) and product 3a (210 mg, 0.36 mmol, 84%) was obtained as a yellow oil.

*2-(4-Ethoxybenzyl)-N,N-diethyl-1-(3-((1,2,3,4-tetrahydroacridin-9-yl)amino)propyl)-1H-benzo[d]imidazole-5-carboxamide (3b).* The reaction was carried out according to general procedure V using 3-(2-(4-ethoxyphenyl)acetamido)-*N*,*N*-diethyl-4-((3-((1,2,3,4-tetrahydroacridin-9-yl)amino)propyl)amino)benzamide 19b (104 mg, 0.17 mmol). The crude product was purified by preparative thin-layer-chromatography (10:1:0.1 CH₂Cl₂:MeOH:NH_{3(aq)} (25%)) and product 3b (23 mg, 39.0 µmol, 23%) was obtained as a yellow oil.

*2-(4-ethoxybenzyl)-N,N-diethyl-1-(4-((1,2,3,4-tetrahydroacridin-9-yl)amino)butyl)-1H-benzo[d]imidazole-5-carboxamide (3c)*. The reaction was carried out according to general procedure V using 3-(2-(4-ethoxyphenyl)acetamido)-*N*,*N*-diethyl-4-((4-((1,2,3,4-tetrahydroacridin-9-yl)amino)butyl)amino)benzamide 19c (166 mg, 0.27 mmol). The crude product was purified by column chromatography (10:1:0.1 CH₂Cl₂:MeOH:NH_{3(aq)} (25%)) and product 3c (46 mg, 76.2 µmol, 28%) was obtained as a yellow oil.

*2-(4-Ethoxybenzyl)-N,N-diethyl-1-(5-((1,2,3,4-tetrahydroacridin-9-yl)amino)pentyl)-1H-benzo[d]imidazole-5-carboxamide (3d).* The reaction was carried out according to general procedure V using 3-(2-(4-ethoxyphenyl)acetamido)-*N*,*N*-diethyl-4-((5-((1,2,3,4-tetrahydroacridin-9-yl)amino)pentyl)amino)benzamide 19d (346 mg, 0.47 mmol). The crude product was purified by column chromatography (10:1:0.1 CH₂Cl₂:MeOH:NH_{3(aq)} (25%)) 3d (22 mg, 35.6 µmol, 51%) was obtained as a yellow oil.

*2-(4-Ethoxybenzyl)-N,N-diethyl-1-(6-((1,2,3,4-tetrahydroacridin-9-yl)amino)hexyl)-1H-benzo[d]imidazole-5-carboxamide (3e).* The reaction was carried out according to general procedure V using 3-(2-(4-ethoxyphenyl)acetamido)-*N*,*N*-diethyl-4-((6-((1,2,3,4-tetrahydroacridin-9-yl)amino)hexyl)amino)benzamide 19e (142 mg, 0.22 mmol). The crude product was purified by preparative thin-layer chromatography (10:1:0.1 CH₂Cl₂:MeOH:NH_{3(aq)} (25%)) and product 3e (70 mg, 0.11 mmol, 50%) was obtained as a yellow oil.

*2-(4-Ethoxybenzyl)-N,N-diethyl-1-(2-(2-(2-((1,2,3,4-tetrahydroacridin-9-yl)amino)ethoxy)ethoxy)ethyl)-1H-benzo[d]imidazole-5-carboxamide (6).* The reaction was carried out according to general procedure V using 3-(2-(4-ethoxyphenyl)acetamido)-*N*,*N*-diethyl-4-((2-(2-(2-((1,2,3,4-tetrahydroacridin-9-yl)amino)ethoxy)ethoxy)ethyl)amino)benzamide 19f (398 mg, 0.58 mmol). The crude product was purified by column chromatography (CH₂Cl₂/MeOH/NH_{3(aq)} (25%) 16:1:0.1) and product 6 (220 mg, 33.1 mmol, 57%) was obtained as a brown oil.

*Ethyl 4-(isopentylamino)-3-nitrobenzoate (20).* 4-Fluoro-3-nitrobenzoic acid 15 (1.00 g, 5.40 mmol) was dissolved in ethanol, a cat. amount (few drops) of H₂SO₄ (95-97%) was added. After stirring overnight under reflux conditions, the mixture was basified with triethylamine and 3-methylbutan-1-amine (691 µl, 5.94 mmol) was added. After stirring under reflux conditions overnight, the mixture was concentrated *in vacuo.* The residue was dissolved in CH₂Cl₂ and washed with 1 m HCl_{(aq)} and a NaHCO₃ solution. The organic layers were combined, dried over anhydrous MgSO₄ and concentrated *in vacuo.* The product 20 (1.46 g, 5.22 mmol, 97%) was obtained as a yellow oil.

*Ethyl 3-amino-4-(isopentylamino)benzoate (21)*. Ethyl 4-(isopentylamino)-3-nitrobenzoate 20 (1.46 g, 5.22 mmol) was dissolved in MeOH and a cat. amount of Pd/C (10%) was added. The mixture stirred under hydrogen atmosphere (8 bar) at room temperature for 3 h. The clear mixture was filtered off by suction over celite. The combined organic layers were dried over anhydrous MgSO₄ and concentrated *in vacuo* and product 36 (1.31 g, 5.22 mmol, quant.) was obtained as a colorless oil. The product was directly used for the next reaction step without further purification.

*Ethyl 3-(2-(4-ethoxyphenyl)acetamido)-4-(isopentylamino)benzoate (22).* The reaction was carried out according to general procedure V using 3 ethyl 3-amino-4-(isopentylamino)benzoate 21 (1.31 g, 5.22 mmol), 2-(4-ethoxyphenyl)acetic acid (1.03 g, 5.74 mmol), HBTU (2.18 g, 5.74 mmol) and NEt₃ (1.08 mL, 7.83 mmol). The product 22 (2.15 g, 5.22 mmol, quant.) was obtained as an orange/brown oil.

*Ethyl 2-(4-ethoxybenzyl)-1-isopentyl-1H-benzo[d]imidazole-5-carboxylate (23).* The reaction was carried out according to general procedure V using ethyl 3-(2-(4-ethoxyphenyl)acetamido)-4-(isopentylamino)benzoate 22 (2.15 g, 5.22 mmol). The crude product was purified by column chromatography (20:1:0.1 CH₂Cl₂/MeOH/NH_{3(aq)} (25%)) and product 23 (1.67 g, 4.23 mmol, 81%) was obtained as a purple oil.

*2-(4-Ethoxybenzyl)-1-isopentyl-1H-benzo[d]imidazole-5-carboxylic acid (24).* Ethyl 2-(4-ethoxybenzyl)-1-isopentyl-1*H*-benzo[*d*]imidazole-5-carboxylate 23 (250 mg, 0.63 mmol) was dissolved in THF, lithium hydroxide (150 mg, 0.63 mmol) in water was added. After 48 h of harsh stirring under reflux conditions, the mixture was concentrated *in vacuo* and the residue was acidified with 2m HCl_{(aq)}. Then, CH₂Cl₂ was added and the organic layer was washed with water. The organic layers were combined, dried over anhydrous MgSO₄ and concentrated *in vacuo.* Product 24 (231 mg, 0.63 mmol, quant.) was obtained as a purple oil.

*2-(4-Ethoxybenzyl)-1-isopentyl-N-(2-((1,2,3,4-tetrahydroacridin-9-yl)amino)ethyl)-1H-benzo[d]imidazole-5-carboxamide (4a).* The reaction was carried out according to general procedure V using 2-(4-ethoxybenzyl)-1-isopentyl-1*H*-benzo[*d*]imidazole-5-carboxylic acid 24 (271 mg, 0.74 mmol), *N*¹-(1,2,3,4-tetrahydroacridin-9-yl)ethane-1,2-diamine 12a (179 mg, 0.74 mmol), HBTU (281 mg, 0.74 mmol) and NEt₃ (145 µL, 1.05 mmol). The crude product was purified by preparative thin-layer chromatography (10:1:0.1 CH₂Cl₂:MeOH:NH_{3(aq)} (25%)) and product 4a (35.0 mg, 59.3 µmol, 8%) was obtained as a yellow oil.

*2-(4-Ethoxybenzyl)-1-isopentyl-N-(3-((1,2,3,4-tetrahydroacridin-9-yl)amino)propyl)-1H-benzo[d]imidazole-5-carboxamide (4b).* The reaction was carried out according to general procedure V using 2-(4-ethoxybenzyl)-1-isopentyl-1*H*-benzo[*d*]imidazole-5-carboxylic acid 24 (370 mg, 1.01 mmol), *N*¹-(1,2,3,4-tetrahydroacridin-9-yl)propane-1,3-diamine 12b (200 mg, 0.78 mmol), HBTU (326 mg, 1.33 mmol) and NEt₃ (184 µL, 1.33 mmol). The crude product was purified by column chromatography (20:1:0.1 CH₂Cl₂:MeOH:NH_{3(aq)} (25%)) and product 4b (67.0 mg, 0.11 mmol, 14%) was obtained as a yellow oil.

*2-(4-Ethoxybenzyl)-1-isopentyl-N-(4-((1,2,3,4-tetrahydroacridin-9-yl)amino)butyl)-1H-benzo[d]imidazole-5-carboxamide (4c)*. The reaction was carried out according to general procedure V using 2-(4-ethoxybenzyl)-1-isopentyl-1*H*-benzo[*d*]imidazole-5-carboxylic acid 24 (286 mg, 0.78 mmol), *N*¹-(1,2,3,4-tetrahydroacridin-9-yl)butane-1,4-diamine 12c (161 mg, 0.60 mmol), HBTU (250 mg, 0.66 mmol) and NEt₃ (141 µL, 1.02 mmol). The crude product was purified by preparative thin-layer chromatography (10:1:0.1 CH₂Cl₂:MeOH:NH_{3(aq)} (25%)) and product 4c (38.0 mg, 0,62 mmol, 10%) was obtained as a yellow oil.

*2-(4-Ethoxybenzyl)-1-isopentyl-N-(5-((1,2,3,4-tetrahydroacridin-9-yl)amino)pentyl)-1H-benzo[d]imidazole-5-carboxamide (4d).* The reaction was carried out according to general procedure V using 2-(4-ethoxybenzyl)-1-isopentyl-1*H*-benzo[*d*]imidazole-5-carboxylic acid 24 (286 mg, 0.78 mmol), *N*¹-(1,2,3,4-tetrahydroacridin-9-yl)pentane-1,5-diamine 12d (170 mg, 0.60 mmol), HBTU (250 mg, 0.66 mmol) and NEt₃ (141 µL, 1.02 mmol). The crude product was purified by column chromatography (20:1:0.1 CH₂Cl₂:MeOH:NH_{3(aq)} (25%)) and product 4d (57.0 mg, 0.32 mmol, 15%) was obtained as a yellow oil.

*2-(4-Ethoxybenzyl)-1-isopentyl-N-(6-((1,2,3,4-tetrahydroacridin-9-yl)amino)hexyl)-1H-benzo[d]imidazole-5-carboxamide (4e)*. The reaction was carried out according to general procedure V using 2-(4-ethoxybenzyl)-1-isopentyl-1*H*-benzo[*d*]imidazole-5-carboxylic acid 24 (114 mg, 0.31 mmol), *N*¹-(1,2,3,4-tetrahydroacridin-9-yl)hexane-1,6-diamine 12e (92.2 mg, 0.31 mmol), HBTU (118 mg, 0.31 mmol) and NEt₃ (65.1 µL, 0.47 mmol). The crude product was purified by column chromatography (20:1:0.1 CH₂Cl₂:MeOH:NH_{3(aq)} (25%)) and product 4e (40.0 mg, 61.9 µmol, 20%) was obtained as a yellow oil.

*2-(4-ethoxybenzyl)-N-ethyl-1-isopentyl-N-(4-((1,2,3,4-tetrahydroacridin-9-yl)amino)butyl)-1H-benzo[d]imidazole-5-carboxamide (5)*. The reaction was carried out according to general procedure V using 2-(4-ethoxybenzyl)-1-isopentyl-1*H-*benzo[*d*]imidazole-5-carboxylic acid 24 (23.7 mg, 64.6 µmol), *N*¹-ethyl-*N*⁴-(1,2,3,4-tetrahydroacridin-9-yl)butane-1,4-diamine 14 (16.0 mg, 53.8 µmol), HBTU (24.5 mg, 64.6 µmol) and NEt₃ (14.9 µL, 0.11 mmol). The solvent was removed *in vacuo,* the residue dissolved in CH₂Cl₂ and washed with NaHCO_{3(aq)}. The crude product was purified by prep. TLC (20:1:0.1 CH₂Cl₂:MeOH:NH_{3(aq)} (25%)). The product 5 (21 mg, 32.5 µmol, 60%) was obtained as a brown oil.

*2-(4-Ethoxybenzyl)-1-isopentyl-N-(2-(2-(2-((1,2,3,4-tetrahydroacridin-9-yl)amino)ethoxy)ethoxy)ethyl)-1H-benzo[d]imidazole-5-carboxamide (7).* The reaction was carried out according to general procedure V using 2-(4-ethoxybenzyl)-1-isopentyl-1*H*-benzo[*d*]imidazole-5-carboxylic acid 24 (40 mg, 0.11 mmol), *N*-(2-(2-(2-aminoethoxy)ethoxy)ethyl)-1,2,3,4-tetrahydroacridin-9-amine 12f (36.2 mg, 0.11 mmol), HBTU (41.7 mg, 0.11 mmol) and NEt₃ (23.6 µL, 0.17 mmol). The crude product was purified by column chromatography (16:1:0.1 CH₂Cl₂:MeOH:NH_{3(aq)} (25%)) and product 7 (33.0 mg 48.7 µmol 44%) was obtained as a brown oil.

*2-(4-Ethoxybenzyl)-1-isopentyl-N-(2-((2-((1,2,3,4-tetrahydroacridin-9-yl)amino)ethyl)disulfaneyl)ethyl)-1H-benzo[d]imidazole-5-carboxamide (8)*. The reaction was carried out according to general procedure V using 2-(4-ethoxybenzyl)-1-isopentyl-1*H*-benzo[*d*]imidazole-5-carboxylic acid 24 (48 mg, 0.13 mmol), *N*-(2-((2-aminoethyl)disulfaneyl)ethyl)-1,2,3,4-tetrahydroacridin-9-amine (35 mg, 0.11 mmol), HBTU (49 mg, 0.13 mmol) and NEt₃ (26 µL, 0.19 mmol). The crude product was purified by preparative thin-layer chromatography (18:1:0.1 CH₂Cl₂:MeOH:NH_{3(aq)} (25%)) and product 8 (35 mg, 51.4 µmol, 47%) was obtained as a yellow oil.

### Inhibition of Cholinesterases

All synthesized compounds were assayed for their ability to inhibit electric eel and human AChE (eeAChE and *h*AChE) and human butyrylcholinesterase (*h*BChE) using the colorimetric Ellman's assay. Results are given in below Table 1. Sequence alignment showed that human AChE (*h*AChE) and the isoform eeAChE exhibit a sequence homology of 88%. Tacrine (compound 1) was selected as reference compound and showed an IC50(*h*BChE) = 12.2 nM and IC₅₀(*h*AChE) = 12.5 nM. The *h*CB₂R agonist compound 2 showed no significant inhibitory activity on cholinesterase (ChE) at 10 µM. However, all synthesized hybrids acted as ChE inhibitors. Compared to tacrine, hybrids 3a, 3b, 3e and 4b showed an increased inhibitory potency towards *h*BChE, while 4b, in addition, also showed higher inhibitory activity towards *h*AChE.

Radioligand binding studies at *h*CB₁R and *h*CB₂R.

All synthesized compounds were tested for affinity to *h*CB₁R and *h*CB₂R using radioligand binding studies. Briefly, *h*CBRs were isolated from cell-lines stably expressing receptors. For *h*CB₂R cells of cell line *HEK h*CB₂R were used. The human embryonic kidney cells (HEK) stably expressing the *h*CB₂R were grown in Dulbecco's modified Eagle's medium containing high glucose supplemented with 8% fetal calf serum and 25 µg/mL zeocin in a 37 °C incubator in the presence of 5% CO₂. Cells were passaged twice a week.

For *h*CB₁R cells of cell line *CHO hCB₁R* were used. The Chinese hamster ovary cells (CHO) stably expressing the *h*CB₁R were grown in Ham's F-12 Nutrient Mix supplemented with 8% fetal calf serum and 400 µg/mL geneticin in a 37 °C incubator in the presence of 5% CO₂. Cells were passaged twice a week.

Synthetic cannabinoid [³H]CP 55,940 that acts as a full agonist at both cannabinoid CB₁ and CB₂ receptors were used as a radioligand. As positive control, the *h*CB₂R selective ligand compound 2 was used. A *K*ᵢ(*h*CB₂R) of 37 nm and selectivity over *h*CB₁R [24% replacement (*h*CB₁R) of radioligand at 10 µM)] was measured. All compounds showed affinity to *h*CB₂R in a single- to two-digit micromolar range. In the case of compounds 3a-e, the affinity towards the *h*CB₂R increased with the length of the spacer from *K*ᵢ = 38.5 µM (3a) to *K*ᵢ = 4.5 µM (3e). Compounds 4a-e all showed a single-digit micromolar affinity towards *h*CB₂R. Experimental data did not support the correlation between affinity and chain length. The tertiary amide derivative compound 5, which is the equivalent of derivative 4c, showed the same affinity towards *h*CB₂R but a higher affinity towards *h*CB₁R resulting in low selectivity. Hence, the number of substituents at the amide position influences selectivity. Hybrids 6 and 7 with a 2-PEG-linker and a resulting higher hydrophilicity and rigidity showed a weaker affinity towards *h*CB₂R compared to the compounds with the longest alkylene linkers (3e and 4e). The disulfide bond in hybrid 8 has no significant effect on affinity towards *h*CB₂R as the equivalent derivative 4e showed almost the same affinity. The results are shown in Table 1. Overall, the linkage of the two molecules has been associated with a loss of affinity at the *h*CB₂R compared to the parent compound 2, but the ligands still retain moderate affinity and good selectivity at *h*CB₂R.

**Table1:**

| Cpd. | R⁵ | R⁶ | R⁷ | IC₅₀ (pIC₅₀ ± SD) or Inhibition [%] | | | *K*ᵢ (pIC₅₀ ± SD) or [³H]CP 55,940 displ. @10 µM | |
|---|---|---|---|---|---|---|---|---|
| | | | | *h*BChE^{a} | AChE | SI^{d} | *h*CB₂R^{e} | *h*CB₁R^{f} |
| Tacrine **1** | | | | 12.2 nM (7.9 ± 0.1) | 18.5 nM^{b} (7.7 ± 0.1) 12.5 nM^{c} (7.9 ± 0.1) | 1.5 | 29% | 24% |
| Rimonabant (inverse agonist for *h*CB₁R) | | | | n.d. | n.d. | n.d. | 4% | 143.0 nM (6.8 ± 0.1) 25.0 nM⁶⁹ |
| SR-144,528 (inverse agonist for *h*CB₂R | | | | n.d. | n.d. | n.d. | 19.7 nM (7.7 ± 0.1) 5.7 nM⁷⁰ | 687.0 nM (6.1 ± 0.1) 264 nM⁷⁰ |
| | 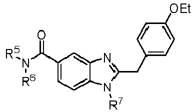 | | | | | | | |
| **2** | 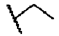 | 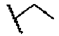 | 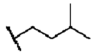 | n.a. | n.a. | n.d. | 36.7 nM (7.4 ± 0.1) 4.5 nM⁵⁶ | 24% >5 µM⁵⁶ |
| **3a** | 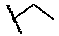 | 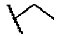 | 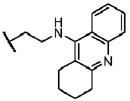 | 1.2 nM (8.9 ± 0.1) | 900 nM^{b} 2.8 nM^{c} (8.6 ± 0.1) | 750 | 38.5 µM (4.4 ± 0.3) | 36% |
| **3b** | 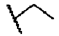 | 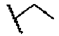 | 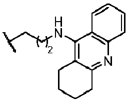 | 2.9 nM (8.5 ± 0.1) | 130 nM^{b} 560 nM^{c} (6.3 ± 0.1) | 44.8 | | 15% |
| **3c** | 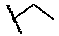 | 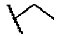 | 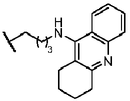 | 33 nM (7.5 + 0.1) | 157 nM^{b} 397 nM^{c} (6.4 ± 0.1) | 4.8 | 26.9 µM (4.6 ± 0.1) | 32% |
| **3d** | 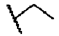 | 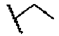 | 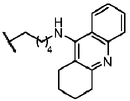 | 5.9 nM (8.2 ± 0.1) | 199 nM^{b} 641 nM^{c} (6.2 ± 0.1) | 33.7 | 7.3 µM (5.1 ± 0.1) | 21% |
| **3e** | 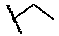 | 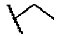 | 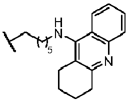 | 1.9 nM (8.7 ± 0.1) | 0.5 nM^{b} 86.1 nM^{c} (7.1 ± 0.1) | 0.3 | 4.5 µM (5.3 ± 0.1) | 17% |
| **4a** | 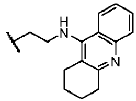 | 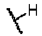 | 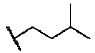 | 81.1 nM (7.1 ± 0.1) | 2.1 nM^{b} 35.6 nM^{c} (7.4 ± 0.1) | 0.0 | 2.5 µM (5.6 ± 0.2) | 5% |
| **4b** | 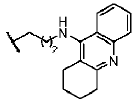 | 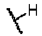 | 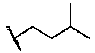 | 3.4 nM (8.5 ± 0.1) | 3.2 nM^{b} 6.03 nM^{c} (8.2 ± 0.1) | 0.9 | 5.0 µM (5.3 ± 0.1) | 20% |
| **4c** | 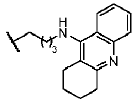 | 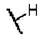 | 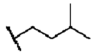 | 10.4 nM (8.0 ± 0.1) | 12.8 nM^{b} 163 nM^{c} (6.8 ± 0.1) | 1.2 | 2.4 µM (5.6 ± 0.4) | 9% |
| **4d** | 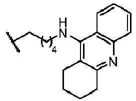 | 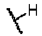 | 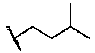 | 8.6 nM (8.1 ± 0.1) | 52.7 nM^{b} 27.0 nM^{c} (6.6 ± 0.1) | 6.1 | 4.0 µM (5.4 ± 0.1) | 15% |
| **4e** | 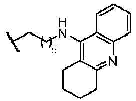 | 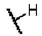 | 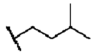 | 18.7 nM (7.7 ± 0.1) | 8.5 nM^{b} 86.5 nM^{c} (7.1 ± 0.1) | 0.5 | 9.6 µM (5.0 ± 0.1) | 54% |
| **5** | 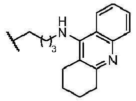 | 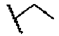 | 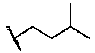 | 60 nM (7.2 ± 0.1) | 370 nM^{b} 259 nM^{c} (6.6 ± 0.1) | 6.2 | 3.0 µM (5.5 ± 0.1) | 36% |
| **6** | 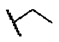 | 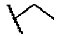 | 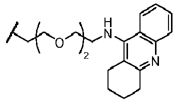 | 90 nM (7.0 ± 0.1) | 150 nM^{b} 390 nM^{c} (6.4 ± 0.1) | 1.7 | 28.7 µM (4.5 ± 0.2) | 11% |
| **7** | 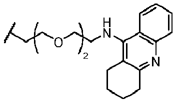 | 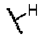 | 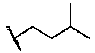 | 370 nM (6.4 ± 0.1) | 1.03 µM^{b} 1.36 µM^{c} (1.4 ± 0.1) | 2.8 | 17.4 µM (4.8 ± 0.1) | 32% |
| **8** | 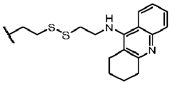 | 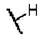 | 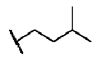 | 77 nM (7.1 ± 0.1) | 44 nM^{b} 13.4 nM^{c} (7.9 ± 0.1) | 0.6 | 6.8 µM (5.2 ± 0.1) | 45% |

| | | | | | | | | |
|---|---|---|---|---|---|---|---|---|
| n.a. activity lower than 10% at the highest tested concentration (10 µM) *^{a}*Values are the means of at least three independent determinations. *h*BChE from human serum. *^{b}*Values are the means of at least three independent determinations. eeAChE from electric eel. *^{c}*Values are the means of at least three independent determinations. *h*AChE recombinant expressed. *^{d}*Selectivity index (SI) towards *h*BChE = IC₅₀(*ee*AChE)/IC₅₀(*h*BChE). *^{e}*Screened on membranes of HEK cells stably expressing *h*CB₂R using 10 µM of the compound, values are mean values from at least 2 independent experiments each performed in triplicates. *^{f}*Screened on membranes of CHO cells stably expressing *h*CB₁R using 10 µM of the compound, values are mean values from at least 2 independent experiments each performed in triplicates. | | | | | | | | |

### Inhibition of amyloid-beta (Aβ) aggregation

For the forty amino acids long isoform A*β*₄₀ of A*β* it is known that aggregation can be induced by AChE whereas it is known for the forty-two amino acids long isoform A*β*₄₂ of Aβ that aggregation of this isoform is self-induced. Inhibitory activity on AChE-induced A*β*₄₀ aggregation was investigated by a Thioflavin T (ThT)-based fluorometric assay and compared with that of the anti-AD drug compound 1. Results shown in Table 2 below demonstrate that compounds 3e and 4b were able to significantly reduce the pro-aggregating action of *h*AChE, while compound 1 was inactive. In the same experimental conditions, the cystamine derivate compound 8 was not able to significantly inhibit the AChE-induced A*β*₄₀ aggregation.

Three selected hybrids were also assayed for their ability to inhibit self-aggregation of A*β*₄₂ by a ThT fluorescence assay. Results are shown in Table 2 below. Interestingly, compounds 3e and 8 strongly inhibited A*β*₄₂ aggregation when assayed at 1/1 ratio with A*β*₄₂ while, in the same conditions, inhibition by 4b was quite weak. The three hybrids showed different inhibition profile towards the two aggregation phenomena. While hybrid 8 is able to significantly interfere with A*β* self-aggregation but not significantly with the chaperonic action of *h*AChE towards A*β*, hybrid 4b is able to significantly interfere with the chaperonic action of *h*AChE towards A*β* but only weakly with A*β* self-aggregation and, finally, hybrid 3e able to significantly inhibit both aggregation phenomena. Because of the strong inhibitory potency towards A*β* self-aggregation exerted by 3e, it cannot be excluded that this activity can also synergistically contribute to the good inhibitory potency recorded in the AChE-induced amyloid aggregation assay. This might also explain the high inhibition value recorded in this assay.

**Table 2:**

| | **% inhibition A*β* aggregation** | |
|---|---|---|
| | **self-induced^{a}** | **AChE-induced^{b}** |
| **3e** | 81.5 ± 02 | 62.9 ± 5.4 |
| **4b** | 16.4 ± 4.2 | 47.8 ± 9.6 |
| **8** | 83.4 ± 6.4 | 5.0 ± 2.6 |
| **1** | <5 | <5 |

| | | |
|---|---|---|
| ^{a}Inhibition of A*β*₄₂ self-aggregation investigated by the thioflavin-T fluorescence assay. Assays were carried out in the presence of 50 µM inhibitor and 50 µM A*β*₄₂ ([I]=[A*β*₄₂]). Values are expressed as mean ± SEM of two independent experiments, each performed in duplicate. ^{b} % inhibition of *h*AChE-induced A*β*₄₀ aggregation at [I] = 100 µM. The A*β*₄₀/*h*AChE ratio was equal to 100/1. Values are expressed as mean ± SEM of two independent experiments, each performed in duplicate. | | |

### Efficacy at hCB₂R

The *h*CB₂R is coupled with G_{i/0} protein, so adenylate cyclase is inhibited in case of activation. This results in a lower level of intracellular cAMP in case of agonist binding. The cAMP increase induced by forskoline (FSK) is enhanced by an antagonist/reverse agonist binding while the binding of an agonist reverts the effect. Going down the signaling pathway mediated by a conformational change of the receptor, the resulting differences in the cellular cAMP level is changing the expression of cAMP-regulated genes (the reporter). A high cAMP level, induced by an antagonist binding leads to a high expression of cAMP-regulated genes, while an agonist leads to the opposite effect. By quantifying the expression of these genes, it is possible to investigate the behavior of the ligand at the receptor. An example of a cAMP-regulated gene is the Macrophage migration Inhibitory Factor (MIF). A further example is the Signal Transducer and Activator of Transcription (STAT-3) gene. Both genes are highly expressed in multiple myeloma cells. Experiments were performed with cells of cell line U266. The U266 cell line was purchased from ATCC (LGC Standards, Milan, IT). The cells were cultured in RPMI1640 medium (Lonza, Milan, IT) supplemented with 10% fetal bovine serum (FBS), 2 mm l-glutamine, 100 IU/mL penicillin, 100 µg/mL ampicillin/streptomycin, 1 mm sodium pyruvate and grown at 37 °C with 5% CO₂ at 95% humidity.

Before examining the efficacy of the compounds 3e and 4c, an MTT assay was carried out on U266 myeloma cells to evaluate a non-toxic concentration of the compounds. The efficacy of the compounds was investigated by the quantification of the cAMP-regulated MIF and STAT-3 genes by a qRT-PCR methodology. AM-630 (6-lodopravadoline) that acts as a potent and selective inverse agonist for the cannabinoid receptor CB₂ was measured as a reference. Cells were treated with compound 2 as a reference agonist, 3e and 4c alone and in combination with AM630. Treatment with test compounds (50 µM), FSK (10 µM) or AM630 (25 µM) occurred for 2 h. In a combined experiment (AM 630 plus test compounds), U266 cells were preincubated with AM630 for 30 min before adding the test compounds. MIF and STAT-3 mRNA levels were determined by qRT-PCR. GAPDH was used for normalization. Results are shown in Fig. 4. Data are expressed as relative fold with respect to vehicle-treated cells used as control. Values are mean of at least two independent experiments, each performed in duplicates. Data are expressed as mean ± SD *p<0.01 vs untreated; # vs AM630.

Compared to the vehicle, compounds 2, 3e and 4c reduced MIF and STAT-3 gene expression. Also, in combination experiments all three compounds show a decreased level of both genes compared to the levels of only AM630 treated cells. The results indicate agonist behavior of compounds 3e and 4c. Because of the structural similarity to the other synthesized compounds, it can be assumed that all hybrids are agonists.

### Effects on microglia activation

For testing a possible immunomodulatory effect, i. e. the shift from the M1 neurotoxic to the M2 neuroprotective phenotype, of compounds 2, 3e and 4a, the murine microglial cell line N9 was used. N9-microglial cells were cultured in Dulbecco Modified Eagle Medium (DMEM) supplemented with 10% heat-inactivated Fetal Bovine Serum (FBS), 1% Penicillin/Streptomycin and 2 mM Glutamine (all cell culture reagents were from Aurogene Srl, Rome, Italy). At confluence, after a short wash with sterile PBS, microglia were trypsinized for 5 min at 37 ° C and trypsin was inactivated with complete DMEM medium. Detached cells were then collected, centrifuged for 5 min at 300xg and resuspended to be counted. For experiments, microglial cells were plated at the density of 2.5x105 in 35 mm Ø dish and exposed to 100 ng/mL lipopolysaccharide (LPS), in presence or absence of increasing concentrations (1 µM, 2.5 µM, 5 µM) of the test compounds. After 24 h of treatment, microglial conditioned media were collected and partly used for nitrite measurement, partly filtered through 0.22-µm filters, concentrated using Microcon YM-3 (Millipore, Billerica, MA) and resuspended in 12 µL of 4× loading buffer (0.2 M Tris-HCI pH 6.8; 8% sodium dodecyl sulfate; 40% glycerol; 0.4% bromophenol blue and 0.4 M dithiothreitol; Sigma-Aldrich) for western blot analysis. In parallel, microglial cells were collected in 2× loading buffer (LB; 50 µL per dish) for western blot analysis.

LPS induces an M1 activation state. Nitrite production due to iNOS induction and IL1β release are markers of M1, neurotoxic activated microglia, while the phagocytic protein TREM2 and TGFβ2 are both makers of M2, neuroprotective microglia.

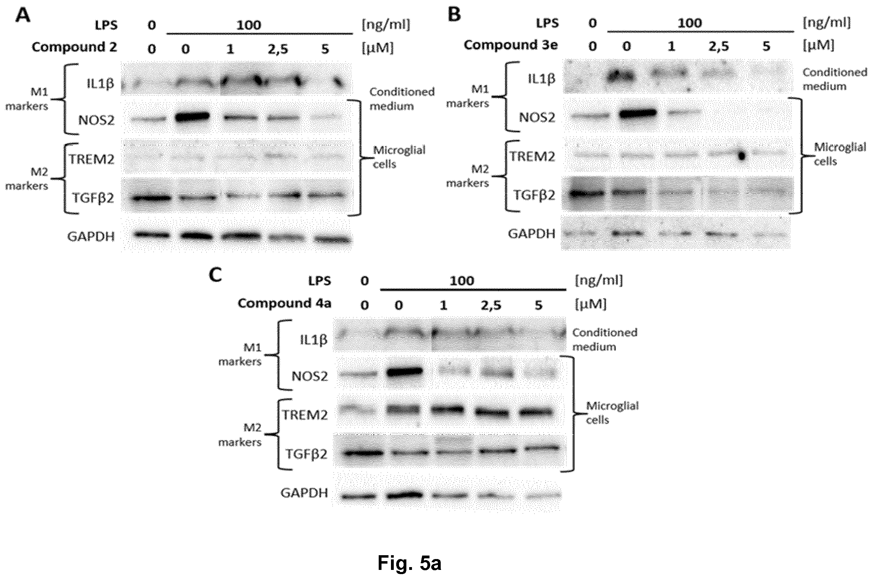

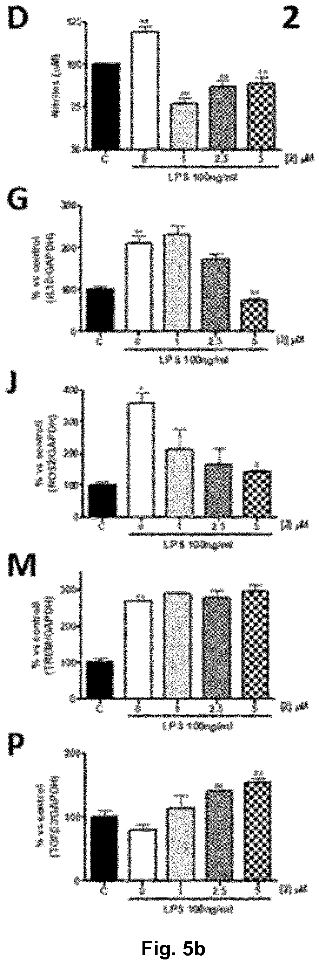

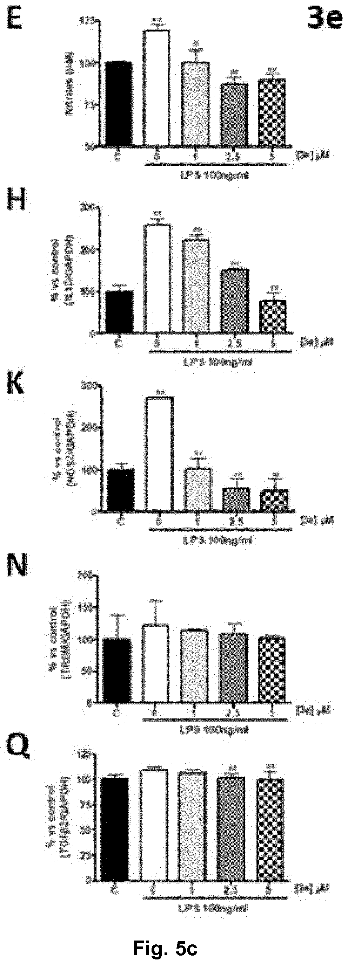

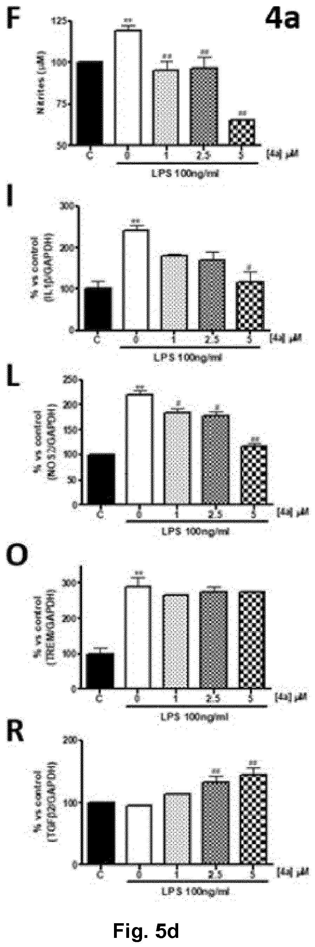

Results are shown in Figs. 5a, 5b, 5c and 5d. Quantification of IL1β release and expression of *i*NOS, TREM2, and TGFβ2 occurred by means of western blot followed by densitometry (G-R). NO release was evaluated by means of colorimetric Griess reaction in media conditioned for 24 h by microglial cells treated by LPS in the presence of compound 2 (D), 3e (E) and 4a (F), showing an increase of NO release in media conditioned by LPS-treated cells that is reduced by the co-treatment with the compounds, with the strongest effect of compound 2. Both IL1β release (G, H, I) and iNOS expression (J, K, L), which are markers of M1 neurotoxic microglia, strongly increase in LPS-treated cells, but significantly decrease in cells co-treated with compound 2 (G, J), 3e (H, K) and 4a (I, L) in a dose-dependent way, while the expression of the M2 microglial markers TREM2 (M, N, O) and TGFβ2 (P, Q, R) is not reduced by co-treatment with the compounds. All quantitative data are presented as means ± S.E.M. from at least 3 independent experiments. Statistical significance between different treatments was calculated by using one-way analysis of variance (ANOVA) followed by post-hoc comparison through Bonferroni's test. *p<0.05; **p<0.01 compare to control, #p<0.05; ##p<0.01 compare to LPS.

As shown in Figs. 5a, 5b, 5c and 5d, compound 3e strongly reduces in a dose-dependent way IL1β release and nitrite accumulation, as well as iNOS expression induced by LPS-mediated activation of microglial cells, with no parallel change in TREM2 and TGFβ2 expression, thus indicating an immunomodulatory effect of the compound, which is similar to compounds 2 and 4a.

### Neuroprotective properties on HT-22 cells

Neuroprotective properties were examined by use of cells of neuronal cell line HT-22 which is derived from murine hippocampal tissue. HT-22 cells were grown in Dulbecco's Modified Eagle Medium (DMEM, Sigma Aldrich, Munich Germany) supplemented with 10% (v/v) heat-inactivated fetal calf serum (FCS) and 1% (v/v) penicillin-streptomycin. Cells were passaged every two days and incubated at 37 °C with 5% CO₂ in a humidified incubator. Compounds were dissolved in DMSO, (Sigma Aldrich, Munich, Germany) and diluted with medium. Generally, 80% confluent cells were seeded with 5000 cells per well into sterile 96-well plates and were incubated for 24 hours.

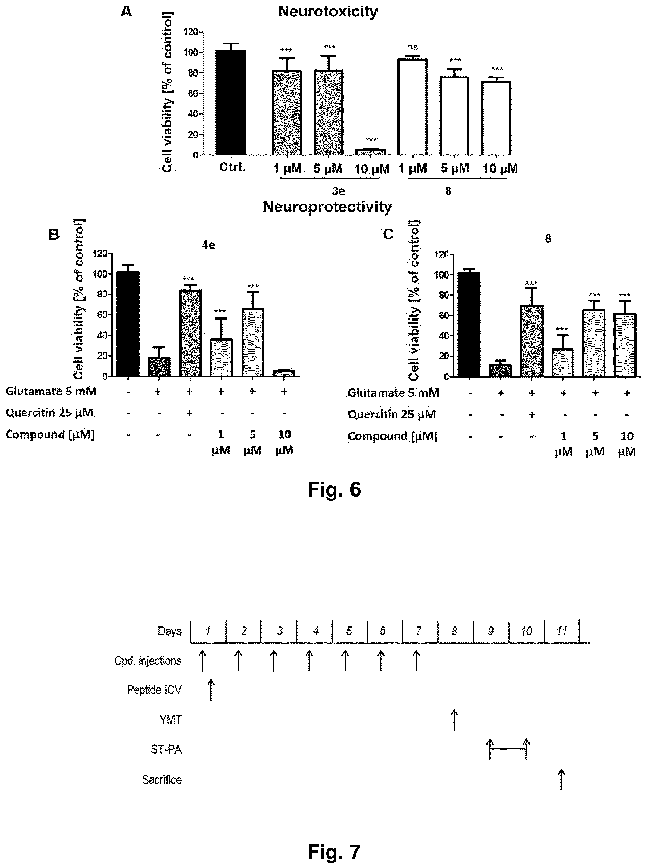

HT-22 cells lack ionotropic glutamate receptors. The addition of extracellular glutamate at high concentrations can be used to introduce intracellular ROS accumulation by blocking the cystine/glutamate antiporter, resulting in glutathione depletion. This neuronal oxidative stress results in cell injuries and eventually cell death. Before performing the neuroprotectivity assays, potential neurotoxicity of both compounds 4e and 8 were evaluated in an MTT assay. For examining neurotoxicity, previous medium was discarded, and the compound in different concentrations was added to the wells. 0.05% DMSO in DMEM served as control. Cells were incubated for 24 hours. After Incubation, MTT-assay was performed. Results are shown in Fig. 6 A.

For examining neuroprotection 5 mM glutamate (monosodium-*I*-glutamate, Sigma Aldrich, Munich, Germany) was co-incubated with different concentrations of respective compounds for 24 hours. 25 µM quercetin (Sigma Aldrich, Munich, Germany) served as positive-control. After 24 h incubation, MTT-assay was performed. Results are shown in Fig. 6 B and C.

Results shown in Fig. 6 A-C are presented as means ± SD of three independent experiments each performed in sextuplicate and refer to untreated control cells which were set as 100% values. Statistical analysis was achieved by applying one-way ANOVA followed by Dunnett's multiple comparison post-test. Levels of significance: *p < 0.005; **p < 0.01; ***p < 0.001. Treated cells were compared to untreated (A) cells and cells treated with glutamate only (B and C).

As can be seen in Fig. 6 C compound 8 showed concentration-dependent neuroprotectivity starting at 1 µM. Treatment with 5 µM of compound 8 leads to neuroprotection comparable to the positive control quercetin at 25 µM. Compound 4e showed a similar neuroprotective behavior but decreases viability at 10 µM due to the neurotoxic effect at this dose (Fig. 6 B). The use of cystamine as a linker yielded a hybrid with lower neurotoxicity compared to the sulfur-free analog 4e with a neuroprotective behavior at a comparable low concentration. Furthermore, compounds 3a, 3d, 3e, 4a, and 4b were also investigated towards their neurotoxicity and neuroprotective behavior. In summary, compounds 3a, 3d and 3e showed no neurotoxicity at the doses tested (max. 10 µM), while 4a is toxic above 5 µM and 4b shows first toxic effects at 5 µM. Compounds 4b and 4a show neuroprotective behavior only at 5 µM, while other compounds tested showed low neuroprotective behavior (3e) or none (3a, 3d).

### In vivo studies

The most promising compounds were tested regarding their pro-cognitive effects in an *in vivo* AD mouse model. To induce AD-like neuroinflammation and cognitive deficits, oligomerized A*β*₂₅₋₃₅ peptide was intracerebroventricularly injected into the mouse brain. Since the addressed targets are located in the brain, hybrids must be able to penetrate the BBB. Due to the high molecular weight, hybrids with longer linkers, such as 3e with a molecular weight of 631, are less likely to cross the BBB. Therefore, compounds 3a and 4a having the shortest spacer lengths but moderate *in vitro* profile were tested first followed by testing of compounds having more pronounced *in vitro* profile. Mice received A*β*₂₅₋₃₅ (9 nmol i.c.v.) or vehicle solution (3 µl i.c.v.) on day 1 and then compounds 3a, 3d, 3e, 4a or 8 in the 0.1-3mg/kg i.p. dose-range, once per day between day 1 to 7. Mice were then tested for spontaneous alternation on day 8 and passive avoidance on days 9-10.

Compounds were solubilized in pure DMSO at a concentration of 2 mg/mL and then diluted in water. Final DMSO concentration in the vehicle solution remained however high (60%) and innocuity was controlled by a daily observation on the mice behavior and weight gain control. None of the treatment affected significantly the weight gain during the week of treatment. Animals lost up to 1 g after the peptide injection but then regained regularly between 0.2 g and 0.4 g daily.

Spatial working memory was evaluated using a spontaneous alternation test in a Y-maze on day 8. The Y-maze is made of grey polyvinylchloride. Each arm is 40 cm long, 13 cm high, 3 cm wide at the bottom, 10 cm wide at the top, and converging at an equal angle. Each mouse was placed at the end of one arm and allowed to move freely through the maze during an 8 min session. The series of arm entries, including possible returns into the same arm, was checked visually. An alternation was defined as entries into all three arms on consecutive occasions. The number of maximum alternations is therefore the total number of arm entries minus two and the percentage of alternation was calculated as (actual alternations / maximum alternations) x 100. Parameters included the percentage of alternation (memory index) and total number of arm entries (exploration index). Animals that showed an extreme behavior (Alternation percentage < 20% or > 90% or a number of arm entries < 10 were discarded from the calculation. In this study, 11 animals were discarded accordingly (3.2% attrition).

Non-spatial long-term memory was analyzed using a step-through passive avoidance test, with training on day 9 and retention on day 10. The apparatus is a two-compartment (15 x 20 x 15 cm high) box with one compartment illuminated with white polyvinylchloride walls and the other darkened with black polyvinylchloride walls and a grid floor. A guillotine door separates each compartment. A 60 W lamp positioned 40 cm above the apparatus lights up the white compartment during the experiment. Scrambled foot shocks (0.3 mA for 3 s) were delivered to the grid floor using a shock generator scrambler (Lafayette Instruments, Lafayette, USA). The guillotine door was initially closed during the training session. During the training session, on day 9, each mouse was placed into the white compartment. After 5 s, the door was raised. When the mouse entered the darkened compartment and placed all its paws on the grid floor, the door was closed, and the foot shock delivered for 3 s. The step-through latency, that is, the time spent to enter the darkened compartment, and the number of vocalizations was recorded. The retention test was carried out 24 h after training, on day 10. Each mouse was placed again into the white compartment. After 5 s, the door was raised. The step-through and escape latencies (escape latency is the time spent to re exit from the darkened compartment) were recorded up to 300 s. Animals that show all latencies during the training and retention session lower than 10 s are considered as failing to learn the task and were discarded from the calculations. In this study, 9 animals were discarded accordingly (2.6% attrition).

A protocol of the experimental treatment and testing of mice is illustrated in Fig. 7. All animals were sacrificed on day 11. The meaning of the abbreviations used in Fig. 7 is as follows: Cpd. Compound, ICV, intracerebroventricular injection; YMT, spontaneous alternation test in the Y-maze; ST-PA, step-through passive avoidance test.

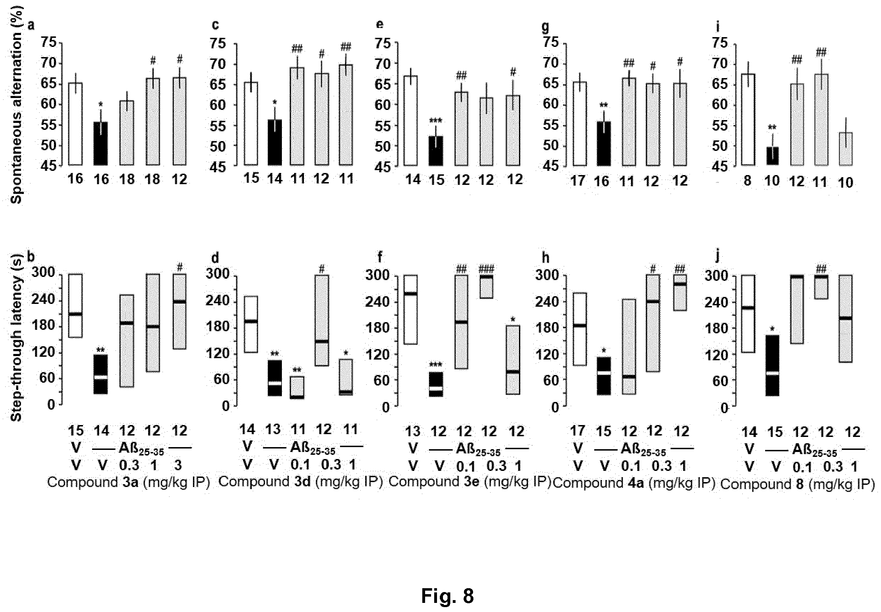

In Fig. 8 a-j shows the effects of the compounds on A*β*₂₅₋₃₅-induced learning impairments in mice. In the upper panels results of spontaneous alternation performance and in the lower panels results of passive avoidance response is shown. Data show mean ± SEM or median (upper panels) and interquartile range (lower panels). ANOVA: *F*_{(4,79)} = 3.05, p < 0.05, n = 12-18 in (a); *F*_{(4,62)} = 3.77, p > 0.01, n = 11-15 in (c); *F*_{(4,64)} = 3.85, p < 0.01, n = 10-15 in (e); *F*_{(4,67)} = 3.14, p < 0.05, n = 11-17 in (g); *F*_{(4,50)} = 5.40, p < 0.01, n = 8-12 in (i). Kruskal-Wallis ANOVA: *H* = 9.87, p < 0.05, n = 12-15 in (b); *H* = 17.4, p < 0.01, n = 11-14 in (d); *H* = 21.9, p < 0.001, n = 12-13 in (f); *H* = 13.9, p < 0.01, n = 12-17 in (h); *H* = 11.8, p < 0.05, n = 12-15 in (j). Post-hoc: *p < 0.05, **p < 0.01, ***p < 0.001 vs. (V+V)-treated group; #p < 0.05, ##p < 0.01, ###p < 0.001 vs. (V+A*β*₂₅₋₃₅)-treated group; Dunnett's (upper panels) or Dunn's test (lower panels).

Results shown in Fig. 8 a - j confirmed that A*β*₂₅₋₃₅ induced significant learning impairments in the behavioral tests. Moreover, all compounds tested showed significant attenuations of A*β*₂₅₋₃₅-induced learning impairments in both the short-term and the long-term memory responses. Effective doses were slightly lower in the spontaneous alternation test (Fig. 8, upper panels) than in the passive avoidance test (Fig. 8, lower panels), and appeared often bell-shaped (Fig. 8 d, f, l, j). Compound 3a significantly prevented A*β*₂₅₋₃₅-induced learning impairments at doses of 1 mg/kg and higher (Fig.8 a, b). All other compounds, namely 3d, 3e, 4a and 8, significantly prevented the learning deficits at the lower dose of 0.3 mg/kg (Fig.8 c-j). Notably, compounds 3e, 4a and 8 completely prevented A*β*₂₅₋₃₅ impairments in both tests at the most effective doses (resp., 0.3, 1 and 0.3 mg/kg), outlining the high efficiency of the compounds. It should be noted at this point that the hybrids show a significantly higher efficacy compared to the parent molecules 1 and 2, which were also investigated *in vivo* as shown in Dolles, D. et al., J. Med. Chem. 2018, 61, pages 1646 to 1663.

### Liver Histology

Livers of mice treated with the high dose (3 mg/kg) were dissected out on day 12, after the behavioral experiments, post-fixed in buffered formalin 4% and cut into 2 mm slices. At least two-third of the parenchyma was embedded, including all macroscopically visible abnormalities. The material underwent standardized dehydration and paraffin embedding as well as hematoxylin-eosin staining after well-established routine protocols. All slides were assessed by the same pathologist, completely blinded to the treatment protocols. The assessment included all typical liver changes seen with drug toxicity: Necrosis was reported focal, segmental, diffuse or zonal in percentages, and divided between fresh and longstanding necrosis. Steatosis was reported as macro- or micro- vesicular, given in percentages, as diffuse or zonal (lobular zones I-III). Fibrosis was assessed according to stages I to IV after Batts and Ludwig. Inflammation is reported semiquantitatively in a four-tiered system 0-3 and divided into portal and/or lobular. Cholestasis was also reported in a four-tiered system 0-3 specified to intrahepatocytic or canalicular. Cell ballooning was also stated in percentage. After the assessment, the findings were realigned with the treating groups. Microvesicular steatosis was present in all groups, including controls (6/10) showing the highest ratio in group 3d (6/6) and the lowest in group 4a (2/9). Necrosis was differentiated into fresh necrosis and older necrosis. The fresh necrosis in these specimen was completely without reaction, only hours old. It was seen in all groups, including controls. Highest ratio was observed in the A*β*/V group (11/12) and group 3d (5/6) with nearly all specimen affected. Older necrosis was reported in two specimen only, both in group A*β*/V. Other findings, like discrete lobular inflammation were seen only in single specimen, not related to treatment groups. The results suggest drug independent reasons for steatosis and fresh necrosis, because they were also found in the control groups in a high percentage. One possible cause of the observed liver damage could be the high level of DMSO used for compound injection. No hepatotoxic relation to compound treatment could be established.

Results of the liver histology performed with high dose (3 mg/kg) treated mice ate shown in table 3 below.

**Table 3:**

| Micro vesicular steatosis | | | | Necrosis | | |
|---|---|---|---|---|---|---|
| Treatment | positive/specimen | % of specimen | reported area % | positive/specimen | % of specimen | reported area % |
| V/V | 6 / 10 | 60 | 30-40 | 5 / 10 | 50 | 2-30 |
| A*β*/V | 7 / 10 | 58 | 10-30 | 11 / 12 | 91 | 1-30 |
| A*β*/3a (3mg/kg) | 8 / 12 | 66 | 5-30 | 8 / 12 | 66 | 2-50 |
| A*β*/3d (3mg/kg) | 6 / 6 | 100 | 3-45 | 5 / 6 | 83 | 5-15 |
| A*β*/4a (3mg/kg) | 2 / 9 | 22 | 10-30 | 6 / 9 | 66 | 5-10 |
| A*β*/8 (3mg/kg) | 8 / 11 | 72 | 20-40 | 8 / 11 | 72 | 5-50 |

### Conclusion

While compound 1 had no effect on A*β* aggregation at the concentration tested, all hybrids tested had an effect. Above all, 3e showed a pronounced inhibition of self and AChE-induced A*β*-aggregation. Radioligand binding studies on *h*CB₂R showed an affinity loss from the nanomolar range of compound 2 to the single-digit micromolar range for the hybrids. Hybrids 3e and 4c, representative for the two different stets, were investigated by a cAMP-regulated gene expression experiment, and the representative compounds maintained their agonist behavior at the *h*CB₂R. To investigate the effectiveness, microglial activity was investigated. Therefore, different markers were quantified. Despite the significantly lower affinity of the hybrids for the *h*CB₂R compared to compound 2, the compounds showed a similar immunomodulatory effect as parent molecule compound 2. The concept of incorporating a disulfide into the linker to introduce neuroprotection was investigated in an HT22 cell assay. Both tested compounds, 8 and the sulfur-free analog 3e showed neuroprotection against glutamate-induced oxidative stress. Due to the promising *in vitro* profile, several compounds were tested *in vivo.* All tested compounds showed pronounced effects regarding short- and long-term memory, proving their ability to cross the BBB. Keeping the hepatotoxicity of compound 1 in mind, liver histology of high dose treated animals (3 mg/kg) was carried out. Tested hybrids showed no hepatotoxic effect. At this point, the high *in vivo* efficacy of the compounds should be emphasized, which is significantly higher in particular for the compounds 3e and 8 (0.1 mg/kg), than for the parent molecules compounds 1 and 2 previously investigated by the inventors. A lower liver-damaging side effect of the novel hybrids may also be due to the significantly lower dosage required to achieve an effect compared to the dosage required of compound 1 according to Dolles, D. et al., J. Med. Chem. 2018, 61, pages 1646 to 1663.

### Abreviations:

A*β*, amyloid-beta; ACh, acetylcholine; AChE, acetylcholinesterase; ACth, acetylthiocholine; AD, Alzheimer's disease; ANOVA, analysis of variance; APP, amyloid precursor protein; BBB, blood-brain-barrier; BChE, butyrylcholinesterase; cAMP, cyclic adenosine monophosphate; CBR, cannabinoid receptor; CB₁R, cannabinoid receptor subtype 1; CB₂R, cannabinoid receptor subtype 2; ChE, cholinesterase; CHO, Chinese hamster ovary; CNS, central nervous system; Cpd, compound; CRE, cAMP response element; CREB, cAMP response-element binding protein; DMSO, dimethyl sulfoxide; Et = ethyl; eeAChE, electric eel AChE; FSK, forskoline; GAPDH, glycerinaldehyde-3-phosphat-dehydrogenase; GTPYS, guanosine 5'[Y-thio]triphosphate; HBTU, 2-(1H-benzotriazol-1-yl)-1,1,3,3-tetramethyluronium hexafluorophosphate; hCB1/2R, human cannabinoid receptor 1/2; HEK, human embryotic kidney; i.c.v., intracerebroventricular; II-1β, interleukin 1 beta; iNOS, inducible nitric oxide synthase; i.p., intraperitoneal; LPS, lipopolysaccharide; MIF, macrophage migration inhibitory factor; MTT, 3-(4,5-dimethylthiazol-2-yl)-2,5-diphenyltetrazolium bromide; NMDA, N-methyl-D-aspartate; PEG, polyethylene glycol; ROS, reactive oxygen species; (q)RT/PCR, (quantitative) reverse transcription polymerase chain reaction; r.t. = room temperature; SD, standard deviation; SR, scavenger receptor; STAT-3, signal transducer and activator of transcription; ST-PA, step-through passive avoidance; TGF β2, transforming growth factor beta-2;THC, tetrahydrocannabinol; THF, tetrahydrofuran; TNF, tumor necrosis factor; TREM2, triggering receptor expressed on myeloid cells 2; V or VEI, vehicle; YMT, Y-maze test

## Claims

1. Compound according to formula 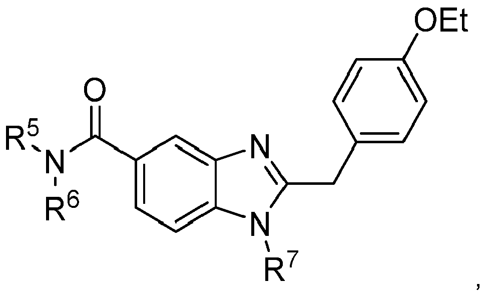 wherein R⁶ is H or an alkyl and one of R⁵ and R⁷ comprises or consists of linker-coupled tacrine residue 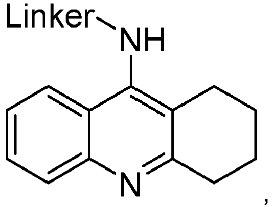 wherein the linker is coupled to the amino residue of the tacrine residue and couples the tacrine residue to the rest of the molecule, wherein the other one of R⁵ and R⁷ is H or an alkyl.

2. Compound according to claim 1, wherein the linker comprises 2 to 8, in particular 2 to 6, C-residues.

3. Compound according to claim 1 or 2, wherein the linker comprises a disulfide group or at least one ether group, in particular two ether groups.

4. Compound according to any of the preceding claims, wherein the alkyl being R⁶ and/or the other one of R⁵ and R⁷ is independent from each other methyl, ethyl, butyl, propyl, pentyl or hexyl.

5. Compound according to any of the preceding claims, wherein the alkyl being the other one of R⁵ and R⁷ is an isoalkyl, in particular isopentyl.

6. Compound according to any of the preceding claims, wherein the compound is 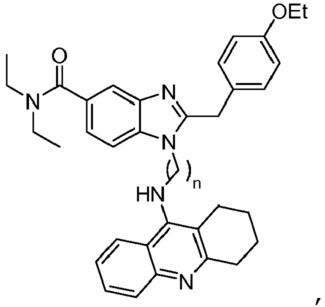 wherein n is 1 to 5 or 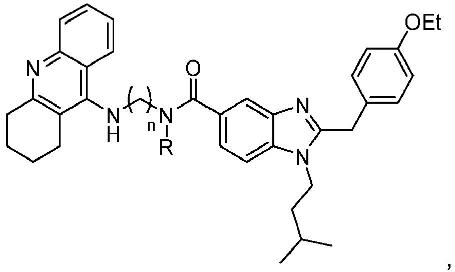 wherein n is 1 to 5 and R is H, methyl, ethyl, butyl, propyl, pentyl or hexyl or 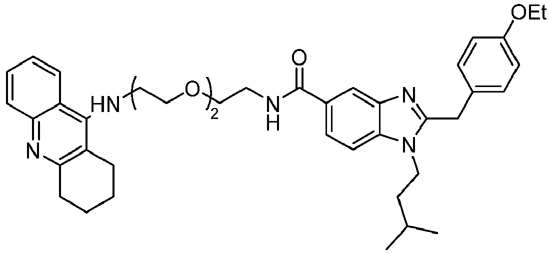 or 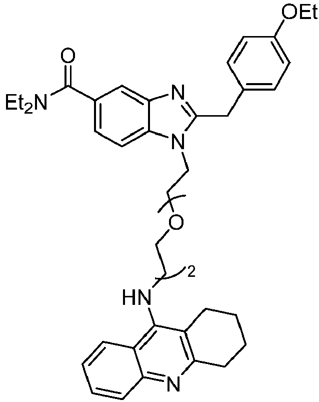 or

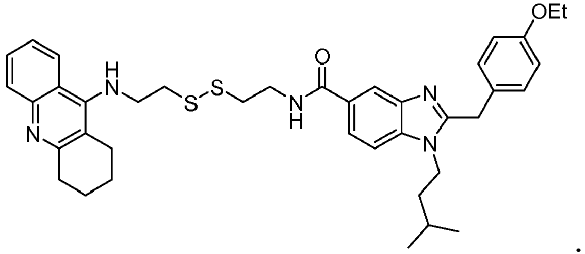

7. Compound according to any of the preceding claims for use in the treatment of Alzheimer's Disease in a mammal.

8. Use of a compound according to any of claims 1 to 6 for inhibition of acetylcholinesterase (AChE) and/or butyrylcholinesterase (BChE) and/or stimulating cannabinoid receptor subtype 2 (CB₂R) *in vitro* or for investigating Alzheimer's Disease in an animal model in which animal model at least one condition of Alzheimer's Disease is artificially induced.

9. Method for synthesizing a compound according to any of claims 1 to 6 comprising the following steps:
a) Reacting anthranilic acid (9) and cyclohexanone (10) with an excess of phosphoroxylchloride to yield chloro-tetrahydroacridine (11),
b) dissolving chloro-tetrahydroacridine (11) and reacting it with a diamine compound to give the corresponding tetrahydroacridine-diamine derivative,
c1) reacting 4-fluoro-3-nitrobenzoic acid (15) with oxalylchloride and a catalytic amount of dimethylformamide (DMF) and then adding diethylamine to yield N,N-Diethyl-4-fluoro-3-nitrobenzamide (16),
d1) reacting N,N-Diethyl-4-fluoro-3-nitrobenzamide (16) with the tetrahydroacridine-diamine derivative of step b) to yield 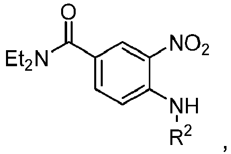 wherein R² is a residue of the tetrahydroacridine-diamine derivative,
e1) reducing the nitro moiety contained in the compound yielded at step d1) by means of a reducing agent to yield corresponding amine compound 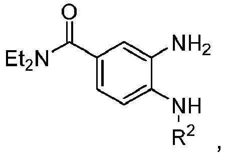
f1) activating 2-(4-Ethoxyphenyl)acetic acid with 2-(1H-Benzotriazol-1-yl)-1,1,3,3-tetramethyluronium-hexafluorophosphat (HBTU) and adding it to the amine compound obtained in step e1) to obtain corresponding amide compound 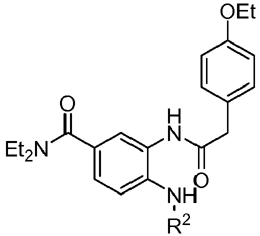 and
g1) cyclisation of the amide compound obtained in step f1) by means of acetic acid to yield compound 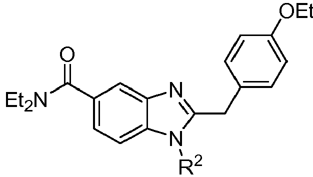 or
c2) adding a catalytical amount of H₂SO₄ to dissolved 4-fluoro-3-nitrobenzoic acid (15) and basifying the resulting mixture after incubation by addition of triethylamine and 3-methylbutan-1-amine to yield ethyl 4-(isopentylamino)-3-nitrobenzoate (20),
d2) dissolving ethyl 4-(isopentylamino)-3-nitrobenzoate (20) and reducing its nitro moiety with hydrogen over Pd/C to yield ethyl 3-amino-4-(isopentylamino)benzoate (21),
e2) activating 2-(4-Ethoxyphenyl)acetic acid with 2-(1H-Benzotriazol-1-yl)-1,1,3,3-tetramethyluronium-hexafluorophosphat (HBTU) and adding it to the ethyl 3-amino-4-(isopentylamino)benzoate (21) to yield ethyl 3-(2-(4-ethoxyphenyl)acetamido)-4-(isopentylamino)benzoate (22),
f2) dissolving ethyl 3-(2-(4-ethoxyphenyl)acetamido)-4-(isopentylamino)benzoate (22) in glacial acetic acid and basifying the resulting reaction mixture after incubation with NH₃ to yield ethyl 2-(4-ethoxybenzyl)-1-isopentyl-1H-benzo[d]imidazole-5-carboxylate (23),
g2) dissolving ethyl 2-(4-ethoxybenzyl)-1-isopentyl-1H-benzo[d]imidazole-5-carboxylate (23) and adding an aqueous lithium hydroxide solution to yield 2-(4-ethoxybenzyl)-1-isopentyl-1H-benzo[d]imidazole-5-carboxylic acid (24) after incubation and
h2) activating 2-(4-ethoxybenzyl)-1-isopentyl-1H-benzo[d]imidazole-5-carboxylic acid (24) with 2-(1H-Benzotriazol-1-yl)-1,1,3,3-tetramethyluronium-hexafluorophosphat (HBTU) and adding the tetrahydroacridine-diamine derivative of step b) to yield 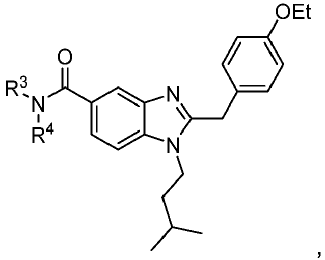 wherein R³ is a residue of the tetrahydroacridine-diamine derivative and R⁴ is H or an alkyl.

10. Method according to claim 9, wherein step b) further comprises acetylating the obtained tetrahydroacridine-diamine derivative for obtaining an amide and reducing this amide to obtain a secondary amine.

11. Method according to claim 10, wherein acetylating is performed by incubation of the tetrahydroacridine-diamine derivative with acetic acid anhydride in dichloromethane and/or reducing is performed by incubation of the amide with LiAlH₄ in dry tetrahydrofuran (THF).

12. Method according to any of claims 9 to 11, wherein the diamine compound is NH₂-(CH₂)₂-NH₂, NH₂-(CH₂)₃-NH₂, NH₂-(CH₂)₄-NH₂, NH₂-(CH₂)₅-NH₂, NH₂-(CH₂)₆-NH₂, NH₂-[(CH₂)₂-O]₂-(CH₂)₂-NH₂ or NH₂-(CH₂)₂-S-S-(CH₂)₂-NH₂.

13. Method according to any of claims 9 to 11, wherein steps a), b), e1), g1), c2) and/or f2) comprise heating and/or step c1) comprises cooling and/or step d2) comprises providing hydrogen at a pressure above atmospheric pressure, in particular above 2 bar.

14. Method according to any of claims 9 to 13, wherein the reducing agent in step e1) is SnCl₂.

15. Method according to any of claims 9 to 14, wherein dissolving in step b) occurs in hexanol, N,N-Diethyl-4-fluoro-3-nitrobenzamide (16) is reacted in step d1) in a solvent, in particular a mixture of tetrahydrofuran (THF) and triethylamine, 4-fluoro-3-nitrobenzoic acid (15) is dissolved in step c2) in ethanol, dissolving of ethyl 4-(isopentylamino)-3-nitrobenzoate (20) in step d2) occurs in methanol, activating of 2-(4-Ethoxyphenyl)acetic acid with 2-(1H-Benzotriazol-1-yl)-1,1,3,3-tetramethyluronium-hexafluorophosphat (HBTU) in step f1) and/or e2) occurs in triethylamine and/or dissolving of ethyl 2-(4-ethoxybenzyl)-1-isopentyl-1H-benzo[d]imidazole-5-carboxylate (23) in step g2) occurs in tetrahydrofuran (THF).
